# EUROPÄISCHE PATENTSCHRIFT

(11) **EP 2 919 829 B1**
(45) Veröffentlichungstag und Bekanntmachung des Hinweises auf die Patenterteilung: **22.02.2017**
(21) Anmeldenummer: 14714664.1
(22) Anmeldetag: 27.03.2014
(51) Int. Cl.: A61M 1/16, A61M 1/34, A61M 1/36, A61B 5/1455

(54) **VORRICHTUNG ZUR IDENTIFIZIERUNG EINER DIALYSATORVORRICHTUNG ODER EINER KOMPONENTE DERSELBEN, UND HIERFÜR VERWENDBARE SENSORVORRICHTUNG**
SYSTEM FOR IDENTIFYING A DIALYZER APPARATUS OR A COMPONENT THEREOF, AND SENSOR DEVICE WHICH CAN BE USED FOR THIS PURPOSE
DISPOSITIF POUR IDENTIFIER UN DISPOSITIF DE DIALYSE OU UN COMPOSANT DE CE DERNIER, ET DISPOSITIF DE DÉTECTION UTILISABLE À CET EFFET

(30) Priorität: 03.04.2013 DE 102013103336
(43) Veröffentlichungstag der Anmeldung: 23.09.2015
(73) Patentinhaber: B. Braun Avitum AG, 34212 Melsungen (DE)
(72) Erfinder: Dr. STROHHÖFER, Christof, 34123 Kassel (DE); KRAUSE, Silvie, 34212 Melsungen (DE)
(74) Vertreter: Winter, Brandl, Fürniss, Hübner, Röss, Kaiser, Polte - Partnerschaft mbB
(86) Internationale Anmeldenummer: PCT/EP2014/056214
(87) Internationale Veröffentlichungsnummer: WO 2014/161772

(56) Entgegenhaltungen:
- WO-A1-02/34314
- DE-A1- 19 627 595
- US-A1- 2006 283 801
- US-A1- 2012 065 568
- US-A1- 2012 095 351
- US-A1- 2012 212 455

## Beschreibung

Die Erfindung betrifft unter anderem eine Vorrichtung zur Identifizierung einer Dialysatorvorrichtung oder einer Komponente derselben wie etwa einer Membranfiltereinrichtung der Dialysatorvorrichtung, z.B. vor oder während des Betriebs der Dialysatorvorrichtung, z.B. bei einer Blutbehandlung eines Patienten. Weiterhin betrifft die Erfindung eine Sensorvorrichtung zur Erfassung eines Zustands und optional zur Identifizierung eines Dialysators, im folgenden auch als Dialysatorvorrichtung bezeichnet, oder einer Komponente hiervon wie etwa einer Membranfiltereinrichtung der Dialysatorvorrichtung; eine Dialysatorvorrichtung, die für das Erfassen oder Messen derartiger Information mittels einer derartigen Sensorvorrichtung ausgebildet ist; ein System, das eine derartige Sensorvorrichtung und eine derartige Dialysatorvorrichtung umfasst; sowie eine Dialysemaschine mit einer derartigen Sensorvorrichtung und einer derartigen Dialysatorvorrichtung.

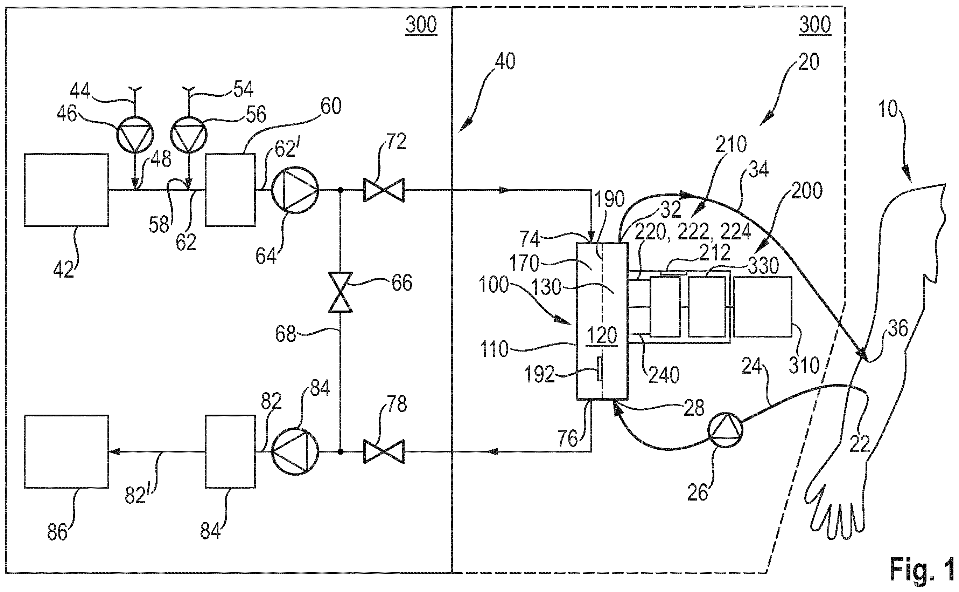

Fig. 1 zeigt eine Dialysemaschine 300 zum Durchführen einer Hämodialysebehandlung eines Patienten 10 in einem Dialysebetrieb. Die Dialysemaschine 300 kann alternativ oder zusätzlich zur Durchführung einer Hämofiltration oder Hämodiafiltration ausgelegt sein, und umfasst ggf. einen Dialysatströmungspfad oder Dialysatflüssigkeitskreislauf 40, hierin auch Dialysatkreislauf 40 genannt, einen in Bezug auf den Patienten 10 extrakorporal geführten Blutströmungspfad oder Blutkreislauf 20 und eine Dialysatorvorrichtung 100 mit einem Innenvolumenbereich 120 und einer darin angeordneten Membranfiltereinrichtung 190 mit einer Membran. Die Dialysatorvorrichtung 100 bewirkt die Realisierung der Blutbehandlung wie etwa eine Hämodialysebehandlung des Patienten 10, weil in ihr bzw. in der darin angeordneten Membran der Stoffaustausch zwischen dem im Blutkreislauf 20 geführten Blut des Patienten 10 und dem im Dialysatkreislauf 40 geführten Dialysat stattfindet. Mit dem Begriff 'Dialysat' ist hier und im folgenden Text die Dialysierflüssigkeit bzw, die verbrauchte Dialysierflüssigkeit bezeichnet.

Die Membran der Membranfiltereinrichtung 190 trennt den Innenvolumenbereich 120 in den vom Blut des Patienten 10 durchströmten Blutbereich 130 und den vom Dialysat durchströmten Dialysatbereich 170 räumlich voneinander. Der Stoffaustausch durch die Membran umfasst den durch einen Konzentrationsgradienten der im Blut gelösten harnpflichtigen, teilweise toxischen Stoffe getriebenen Durchtritt derselben durch die Membran in das Dialysat und umgekehrt den durch einen Konzentrationsgradienten der im aufbereiteten Dialysat gelösten Stoffe getriebenen Durchtritt derselben durch die Membran in das Blut des Patienten. Die im aufbereiteten Dialysat enthaltenen Stoffe werden von Komponenten 42, 44, 54 der Dialysataufbereitung in einer für den Patienten 10 spezifisch aufbereiteten Konzentrationsverteilung in das Dialysat eingebracht und gelöst.

Es sind Einrichtungen bekannt, mit deren Hilfe außerhalb der Dialysatorvorrichtung 100 Messungen von Parametern vorgenommen werden können, aus welchen Parametern auf die Leistungsfähigkeit, z.B. die Dialysance der Dialysatorvorrichtung bzw. die Clearance des extrakorporalen Blutkreislaufs geschlossen werden kann. Ebenfalls sind entsprechende Verfahren zum Auswerten der von den Einrichtungen gemessenen Parameter und zum Bereitstellen von Information über die Leistungsfähigkeit einer Dialysatorvorrichtung bekannt.

Die US 2012/095351 A1 offenbart ein Gerät zur Behandlung von Blut in einem extrakorporalen Kreislauf. Es weist zumindest eine Blutpumpe und zumindest eine Filtereinheit auf.

Das Gerät enthält zumindest ein Druckmesselement zur Erkennung des Blutdrucks, wobei das Druckmesselement eine Sensoreinheit und einen RFID-Transponder aufweist.

Die WO 02/34314 A1 offenbart ein integriertes Einwegset zur Dialyse oder Ultrafiltrationsbehandlung von Blut und enthält einen Speicherchip. Dieser Speicherchip enthält Informationen, die sich auf das Wegwerfset bzw. das Einwegset beziehen. Die Daten schließen beispielsweise Kalibrationsdaten für die Sensoren (Modell, Datum der Herstellung und andere Informationen) ein.

Die US 2006/283801 A1 bezieht sich auf eine Methode und ein Gerät zur Zubereitung von Flüssigkeiten, die in einem Gerät zur extrakorporalen Blutbehandlung genutzt werden, und auf ein Gerät für Behandlung von Blut, welche die genannte Methode und das genannte Gerät verwendet.

Die DE 196 27 595 A1 offenbart einen Filter, der eine Filterpatrone mit einer Speichereinrichtung umfasst, die elektronisch lesbare Information enthält, die von einem Sensor empfangen wird. Diese Information kann von einem externen Lesegerät, z.B. über einen Sender oder einen Ausgabeschluss gelesen werden.

Die US 2012/212455 A1 offenbart ein technisches medizinisches Gerät mit einem multifunktionalen Bildschirm. Der Bildschirm kann als Lautstärkenumwandler, als optischer Sensor oder als Sender und Empfänger für elektromagnetische Wellen agieren. Des Weiteren wird ein Verfahren offenbart, bei dem die Kontrolle des technischen medizinischen Geräts durch die Ein- und Ausgabe von Informationen über den Bildschirm beeinflusst werden kann.

Die US 2012/065568 A1 offenbart An extracorporeal blood chamber for an optical blood monitoring system has a mixing area and viewing area that are offset from the axis of the blood flow path into and out of the blood chamber. The off-axis design provides more leverage for threading the blood chamber onto a dialysis filter. It also eliminates the need for turbulence posts

DE 32 23 051 A1 offenbart eine Dialysevorrichtung mit geregelter Dialysatzusammensetzung, wobei die stoffliche Zusammensetzung des Dialysats auf der Grundlage der Signale von stromauf und stromab des Dialysatbereichs im Dialysatkreislauf eingeschlauchter Sensoren zum Messen der elektrischer Leitfähigkeit bestimmt wird. Auf der Grundlage der in DE 32 23 051 A1 offenbarten Einrichtung kann mittels eines in EP 428 927 A1 offenbarten Verfahrens auf der Grundlage einer Variation der Zusammensetzung der Dialysatflüssigkeit die Dialysance bestimmt werden. Der mit diesem Verfahren für die Dialysance des Dialysators ermittelte Zahlenwert ist identisch zu dem der Clearance, die die aus dem Blut entfernte Menge von z. B. toxischen Stoffen pro Zeiteinheit angibt.

DE 10 2010 043 574 A1 offenbart einen manuell zu öffnenden Klemmhalter zur lösbaren Befestigung an einem medizinischen Einmalartikel, wie etwa einer Leitung bzw. eines Schlauchs des Blutkreislaufs 20 oder eines als Einmalartikel ausgebildeten Dialysators 100. Der Klemmhalter umfasst eine erste Klemmbacke und eine daran angelenkte und in Bezug auf die erste Klemmbacke verschwenkbare zweite Klemmbacke, zwischen denen der zu befestigende Einmalartikel eingeklemmt werden kann, und einen Sensor zum Erfassen der relativen Stellung der Klemmbacken. Aus dem von diesem Sensor erzeugten Signal kann auf die relative Stellung der Klemmbacken und mithin auf die Größe bzw. einen Durchmesser des zwischen den Klemmbacken eingeklemmten Gegenstands geschlossen werden. Daraus wird auf die Art bzw. einen Typ des eingeklemmten Gegenstands geschlossen.

US 2012/0154789 A1 beschreibt eine Sensorclip-Einrichtung zur optischen Überwachung von durch eine in-line in einer Blutleitung eingeschlauchte Blutkammer hindurchströmendem Blut, insbesondere von im Blut gelösten Stoffen. Die Sensorclip-Einrichtung umfasst zwei relativ zueinander verschwenkbare und bezüglich der Blutkammer einander gegenüberliegend angeordnete Klemmbacken, zwischen denen die Blutkammer eingeklemmt werden kann. In der ersten Klemmbacke ist ein Lichtemitter und in der zweiten Klemmbacke ein Fotodetektor angeordnet. Der Fotodetektor misst die Intensität von Licht mit Wellenlängen im infraroten Spektralbereich, das das durch die Blutkammer strömende Blut durchlaufen hat. Die Wellenlängen sind stoffspezifisch selektiv so ausgewählt, dass die Absorption des Lichts an spezifischen, im durchströmenden Blut enthaltenen Stoffen, wie etwa Hämatokrit oder Sauerstoff, gemessen und Veränderungen im Blutvolumen bestimmt werden können.

Die Erfindung wird durch die Merkmale des unabhängigen Anspruchs 1 definiert.

Die Erfindung beruht unter anderem auf der Erkenntnis, dass eine berührungslose Messung bezüglich einer Identifizierung, beispielsweise eines Typs, einer Dialysatorvorrichtung bzw. einer Komponente der Dialysatorvorrichtung , beispielsweise einer in der Dialysatorvorrichtung angeordneten Membranfiltereinrichtung vorteilhaft ist. Mit Ausführungsbeispielen der Erfindung kann direkt am Dialysator gemessen oder eine Identifizierung durchgeführt werden. Dies ist vorteilhaft, weil der Dialysator aus therapeutischer Sicht den kritischsten Teil einer Dialysemaschine darstellt, weil dort der Stoffaustausch stattfindet und dort die Dialyseeffizienz wesentlich beeinflusst wird. Auch steht im Dialysator aufgrund der Geometrie das Blut in starker Wechselwirkung mit der Membran der Membranfiltereinrichtung, so dass die stärksten Effekte auf blut- bzw. dialysatseitige Parameter gerade im Dialysator stattfinden. Hierbei kann auch berücksichtigt werden, dass sich für die Dialyseeffizienz indikative Parameter während der Verweilzeit des Bluts im Dialysator verändern können, wie etwa Hämatokrit und Koagulationseigenschaften des Bluts, die außerhalb des Dialysators durch Kompensationsmechanismen wieder in ihren Ursprungszustand zurückkehren. Beispielsweise nimmt der Hämatokrit aufgrund der Druckverhältnisse in modernen, auf hohen Durchfluss hin optimierten Dialysatoren über die Länge des Dialysators, insbesondere über die Länge des Blutbereichs, zunächst zu und dann wieder ab. Dieser Verlauf der Hämatokritkonzentration im Dialysator kann bisher nicht direkt gemessen werden, obwohl bekannt ist, dass die maximale Hämatokritkonzentration ein entscheidender Parameter für Koagulations-, Verclottungs- und Hämolyseeffekte ist. Auch enthalten lokale Konzentrationen und Konzentrationsunterschiede der im Dialysat gelösten Stoffe wichtige Informationen über die Qualität der Blutreinigung im Dialysator entlang der Länge der Membran. Eine bei Ausführungsbeispielen vorgesehene Messung bzw. Überwachung entsprechender Parameter, wie z.B. beispielsweise des Koagulationszustands, direkt am Dialysator ist daher vorteilhaft.

Mit der Erfindung wird gemäß einem Aspekt eine Vorrichtung zur Identifizierung einer Dialysatorvorrichtung oder einer Komponente derselben bereitgestellt. Hierbei kann eine wieder verwendbare Sensorvorrichtung bzw. ein System mit einer wieder verwendbaren Sensorvorrichtung eingesetzt werden, die bzw. das es ermöglicht, möglichst direkte Informationen bezüglich einer Identifizierung einer z.B. als Einmalartikel ausgebildeten Dialysatorvorrichtung oder einer z.B. als Einmalartikel ausgebildeten Membranfiltereinrichtung der Dialysatorvorrichtung direkt an der Dialysatorvorrichtung zu erfassen.

Gemäß einer oder mehreren Ausführungsformen wird eine Vorrichtung zum Messen von Parametern bereitgestellt, aus denen Information bezüglich einer Identifizierung einer Dialysatorvorrichtung oder einer Membranfiltereinrichtung der Dialysatorvorrichtung während ihres Betriebs in einer Dialysebehandlung eines Patienten gewonnen werden können. Dabei werden die Messungen direkt an der Dialysatorvorrichtung vorgenommen. Die Dialysatorvorrichtung bzw. zumindest die darin eingesetzte Membranfiltereinrichtung ist in der Regel ein Einmalprodukt, das nur während einer einzigen Dialysebehandlung an einem Patienten genutzt wird und danach entsorgt wird. Daher ist die Wiederverwendbarkeit der Sensoreinrichtung für mehrere Dialysatorvorrichtungen vorteilhaft.

Mit der Erfindung werden eine Vorrichtung mit den Merkmalen des Patentanspruches 1, sowie eine Sensorvorrichtung, eine Dialysatorvorrichtung und eine Dialysemaschine geschaffen.

Gemäß einem ersten Aspekt der Erfindung wird eine Vorrichtung zum Erfassen oder Messen von Informationen zur Identifizierung einer Dialysatorvorrichtung oder einer Komponente wie etwa einer Membranfiltereinrichtung der Dialysatorvorrichtung vor und/oder während des Betriebs der Dialysatorvorrichtung in einer Dialysebehandlung eines Patienten bereitgestellt. Das System umfasst bei einem oder mehreren Ausführungsbeispielen eine Dialysatorvorrichtung mit einem einen Innenvolumenbereich umschließenden Gehäuse und einer im Wesentlichen in dem Innenvolumenbereich angeordneten Membranfiltereinrichtung, und eine Sensorvorrichtung, die mit dem Gehäuse der Dialysatorvorrichtung lösbar operativ verbindbar ist und die eine Signalempfangseinrichtung umfasst, die dazu ausgebildet ist, mindestens ein Signal, wie etwa ein Strahlungssignal, z.B. von der Gehäuseoberfläche, von einem an oder in dem Gehäuse angebrachten oder ausgebildeten Identifikationsträger wie etwa einem Etikett, oder aus dem Innenvolumenbereich des Gehäuses zu empfangen, wobei das Signal charakteristisch für den Zustand bzw. die Identifizierung der Dialysatorvorrichtung bzw. der Membranfiltereinrichtung der Dialysatorvorrichtung ist. Die Sensorvorrichtung kann mit einem Gehäuse (110) der Dialysatorvorrichtung (100) derart lösbar verbindbar sein, dass sie von dem Gehäuse gehalten wird. Alternativ kann die Sensorvorrichtung auch so ausgebildet sein, dass sie das Gehäuse hält, wobei sie z.B. in den Halter für das Dialysatorgehäuse integriert sein kann oder selbst an diesem angebracht sein kann.

Das Gehäuse kann optional für ein Signal, wie etwa ein Strahlungssignal, zumindest bereichsweise durchlässig sein, um z.B. Materialien, Substanzen oder Markierungen am oder im Inneren des Gehäuses erkennen zu können. Eine Identifizierung des Dialysators oder von Komponenten desselben kann alternativ oder zusätzlich mittels außen am Gehäuse angebrachter Merkmale bewerkstelligt werden.

Gemäß einem zweiten Aspekt der Erfindung wird eine Sensorvorrichtung bereitgestellt, die mit einem Gehäuse einer Dialysatorvorrichtung lösbar operativ verbindbar ausgebildet ist.

Gemäß einem dritten Aspekt der Erfindung wird eine Dialysatorvorrichtung, also vorzugsweise ein Dialysator, mit einem einen Innenvolumenbereich umschließenden Gehäuse und einer im Wesentlichen in dem Innenvolumenbereich angeordneten Membranfiltereinrichtung bereitgestellt, wobei das Gehäuse für ein Signal, wie etwa ein Strahlungssignal, durchlässig, undurchlässig oder zumindest bereichsweise durchlässig sein kann. Die Dialysatorvorrichtung kann hierbei so ausgebildet sein, dass mit ihr eine Sensorvorrichtung lösbar operativ verbindbar ist.

Gemäß einem vierten Aspekt der Erfindung wird eine Dialysemaschine zum Durchführen einer Dialysebehandlung an einem Patienten bereitgestellt. Die Dialysemaschine umfasst eine Dialysatorvorrichtung gemäß dem dritten Aspekt der Erfindung und eine Sensorvorrichtung gemäß dem zweiten Aspekt der Erfindung. Die Sensorvorrichtung kann mit der Dialysatorvorrichtung lösbar operativ verbunden sein oder werden.

Die mit der Dialysatorvorrichtung lösbare und operativ verbindbare Ausgestaltung der Sensorvorrichtung ermöglicht, dass die Sensorvorrichtung wiederholt bzw. an mehreren Dialysatorvorrichtungen zum Messen der Information verwendet werden kann, während die Dialysatorvorrichtung oder Teile davon als Einmalartikel ausgebildet sein können.

Die direkte Ankoppelbarkeit der Sensorvorrichtung an der Dialysatorvorrichtung, d.h. also am Dialysator, ermöglicht, dass die Messung von Informationen direkt an der Dialysatorvorrichtung durchgeführt werden kann und so die gewünschte Information möglichst direkt bzw. detailliert gemessen werden kann.

Die erfindungsgemäße Sensorvorrichtung ermöglicht, eine Identifizierung der Dialysatorvorrichtung oder der Komponente, z.B. der Membranfiltereinrichtung der Dialysatorvorrichtung, direkt, fehlerfrei und zuverlässig zu erkennen bzw. Informationen bezüglich des Zustands der Dialysatorvorrichtung, insbesondere des Zustands der Komponente, z.B. der Membranfiltereinrichtung präzise, detailliert und direkt an der Dialysatorvorrichtung bzw. der Komponente, z.B. der Membranfiltereinrichtung zu messen. Derartige Informationen können bezüglich der Effizienz des Stoffaustauschs durch die Membran der Membranfiltereinrichtung indikative Parameter, einschließlich insbesondere der Dialysance umfassen. Die Stoffaustauscheffizienz ist abhängig von der stofflichen Zusammensetzung des aufbereiteten Dialysats, von der stofflichen Zusammensetzung, insbesondere der Konzentrationen von im Blut des Patienten enthaltenen und durch die Dialysebehandlung dem Blut bzw. dem Patienten zu entziehenden Stoffen, und von Vorgängen in der Dialysatorvorrichtung insbesondere auf kapillarem Niveau bzw. in den Poren der Membran der Membranfiltereinrichtung, wie etwa eine z.B. lokalisierte Verstopfung der Membran durch z.B. Filterverclottung. Das zu messende Signal kann so ausgewählt werden, dass es für mindestens eine dieser Größen indikativ ist. Hierbei besteht die Möglichkeit einer ortsaufgelösten Messung von Parametern im Dialysator. Die Messung kann hierbei gleichzeitig oder sequentiell an zwei oder mehr Stellen am oder im Dialysator erfolgen, z.B. entlang des Dialysators in dessen Achsrichtung, entlang seines Umfangs oder auch schräg hierzu z.B. entlang einer oder mehrerer Linien oder an verteilten Orten in sonstiger beliebig verteilter Weise, oder auch an mehreren, zumindest zum Teil in der Radialrichtung des Dialysators aufeinanderfolgend angeordneten Messstellen.

Die erfindungsgemäße Vorrichtung kann dazu ausgebildet sein, das gemessene Signal einer Auswerteeinrichtung bereitzustellen, die auf der Grundlage des gemessenen Signals die Dialysatorvorrichtung identifiziert oder zumindest identifizieren kann und ggf. zusätzlich Daten erzeugt, die zum Steuern von Betriebsparametern einer Dialysemaschine verwendet werden können.

Die Signalempfangseinrichtung kann dazu ausgebildet sein, Signale einer Strahlung zu detektieren, die beliebiger Art sein kann oder auch aus einer Gruppe ausgewählt sein kann, die z.B. folgendes umfasst: a) elektromagnetische Strahlung mit einer beliebigen Wellenlänge, z.B. im optischen Bereich wie etwa im Bereich fernen Infrarot (FIR), infraroten (IR), nahinfraroten, sichtbaren und ultravioletten (UV) Lichts, b) elektromagnetische Strahlung mit einer Wellenlänge oder einem Wellenlängenbereich bzw. der entsprechenden Frequenz oder dem entsprechenden Frequenzbereich aus dem gesamten elektromagnetischen Spektrum wie etwa z.B. im Mikrowellenbereich, im Terahertzbereich, d.h. im Submillimeterstrahlungsbereich, oder im Bereich von Radiowellen, wie etwa in der RFID-Technologie verwendeten Radiowellen, und c) Ultraschall-Strahlung. Die vielseitige Ausgestaltbarkeit der Signalempfangseinrichtung ermöglicht, dass unterschiedliche Informationen bzw. Arten von Informationen bzw. verschiedenartige Parameter der Dialysatorvorrichtung oder der Membranfiltereinrichtung gemessen werden können.

Die Signalempfangseinrichtung kann einen Empfänger für ein magnetisches, elektrisches oder elektromagnetisches Signal umfassen, wobei das Signal indikativ für eine zu messende Kapazität und/oder eine zu messende Induktivität ist, die charakteristisch für den Zustand bzw. die Identifizierung der Dialysatorvorrichtung und/oder der Membranfiltereinrichtung ist. Ein solches Signal kann z.B. störungsfrei und zuverlässig beispielsweise vom Außenbereich oder auch aus dem Rand- oder Innenvolumenbereich der Dialysatorvorrichtung von dem z.B. an oder außerhalb der Dialysatorvorrichtung befindlichen Empfänger erfasst werden.

Die Sensorvorrichtung gemäß dem zweiten Aspekt der Erfindung kann eine Strahlungsaussendeeinrichtung umfassen, die dazu ausgebildet ist, Strahlung, insbesondere Strahlung aus dem gesamten elektromagnetischen Spektrum wie etwa Strahlung mit einer Wellenlänge oder einem Wellenlängenbereich bzw. der entsprechenden Frequenz oder dem entsprechenden Frequenzbereich aus dem gesamten elektromagnetischen Spektrum wie etwa z.B. im Mikrowellenbereich, im Terahertzbereich, d.h. im Submillimeterstrahlungsbereich, oder im Bereich von Radiowellen, wie etwa in der RFID-Technologie verwendeten Radiowellen, oder optische Strahlung, oder Ultraschall-Strahlung, auf das Gehäuse oder auch in den Innenvolumenbereich des Gehäuses der Dialysatorvorrichtung auszusenden. Entsprechend kann die Signalempfangseinrichtung dazu ausgebildet sein, die Intensität von Strahlung, die von der Strahlungsaussendeeinrichtung ausgesendet worden ist, zu messen. Dabei kann im Betrieb die gemessene Strahlung das Ergebnis einer für den Zustand der Dialysatorvorrichtung charakteristischen Wechselwirkung sein, die im Innenvolumenbereich des Gehäuses zwischen der Strahlung und einem oder mehreren der folgenden stattgefunden hat: dem Dialysat bzw. dem Blut, einem in dem Dialysat und/oder in dem Blut enthaltenen Stoff, der Membranfiltereinrichtung und einem in bzw. an der Membranfiltereinrichtung festgehaltenen Stoff, der aus dem Blut bzw. dem Dialysat entstammt. Dabei kann zumindest ein Teil der gemessenen Strahlung aus dem Innenvolumenbereich zu der Signalempfangseinrichtung gelangen. Insbesondere kann dabei die Wechselwirkung durch von der Strahlungsaussendeeinrichtung in den Innenvolumenbereich des Gehäuses ausgesendete Strahlung hergerufen worden sein. Das Messen eines Ergebnisses einer elektromagnetischen, z.B. optischen, Wechselwirkung von z.B. in den Innenvolumenbereich eingestrahlter Strahlung ermöglicht berührungslose Messungen an den genannten, z.B. im Innenvolumenbereich im Betrieb vorhandenen Elementen und damit Messungen von direkt den Zustand der Dialysatorvorrichtung charakterisierenden Parametern. Bei der Messung von Strahlung, die zum Beispiel von der Oberfläche oder aus dem Innenbereich des Dialysators stammt und als Reflexion, Streuung, Beugung oder als Frequenzumsetzung von diffuser oder gezielt ein- oder aufgestrahlter elektromagnetischer Strahlung wie etwa Licht erhalten wird, kann bei einem, mehreren oder allen Ausführungsbeispielen eine ortsaufgelöste Messung durchgeführt werden. Ortsaufgelöste Messungen ermöglichen beispielsweise die ortsaufgelöste Erfassung dialysatorspezifischer Eigenschaften. Der Einsatz der Ortsauflösung als Messprinzip erlaubt es z.B., die Beeinflussung wie etwa Beugung oder Ablenkung eines einfallenden Lichtstrahls zu bestimmen und daraus Parameter abzuleiten. Zur Ortsauflösung kann z.B. an einer oder mehreren Positionen gemessen werden, so dass dialysatorspezifische Eigenschaften ortsaufgelöst gemessen werden können. Hierbei kann die Mehrzahl von Messungen zur Erhöhung der Messgenauigkeit miteinander verrechnet werden. Dazu kann die erfasste Strahlung an mehreren Punkten in Richtung, oder quer, oder schräg zu der Strahlungsaus- oder -eintrittsrichtung durch einen oder mehrere Sensoren detektiert werden. Damit ergibt sich eine Ortsauflösung als Messprinzip, z.B. um die Ablenkung einer einfallenden elektromagnetischen Strahlung wie etwa von Licht zu bestimmen und daraus Parameter, z.B. über den Brechungsindex, den Innenaufbau, das Material des Dialysators oder dgl. abzuleiten.

Es kann eine elektromagnetische, z.B. optische, Wechselwirkung hervorgerufen werden, die z.B. aus einer Gruppe ausgewählt ist, die folgendes umfasst:
- Reflektion von Strahlung an der Gehäuseoberfläche, oder von der Strahlungsaussendeeinrichtung ausgesendeter elektromagnetischer, z.B. optischer, Strahlung an einer Grenzfläche zwischen der Gehäusewand, beispielsweise dem Fensterbereich, und dem Dialysat oder dem Blut,
- Reflektion von von der Strahlungsaussendeeinrichtung ausgesendeter Strahlung an einer Grenzfläche zwischen der Membranfiltereinrichtung und dem Dialysat oder dem Blut,
- Transmission von von der Strahlungsaussendeeinrichtung ausgesendeter Strahlung mit einer Messwellenlänge, die von einem in dem Dialysat und/oder in dem Blut enthaltenen Stoff absorbiert werden kann und die auf ihrem Weg zu der Strahlungsempfangseinrichtung einen Strahlungslaufweg durch das Dialysat und/oder durch das Blut zurückgelegt hat,
- Emission von Lumineszenz- oder Fluoreszenzstrahlung durch einen in dem Dialysat und/oder in dem Blut enthaltenen Stoff, wobei eine Lumineszenz- oder Fluoreszenzreaktion in diesem Stoff durch von der Strahlungsaussendeeinrichtung ausgesendete Strahlung hervorgerufen worden ist,
- Brechung von von der Strahlungsaussendeeinrichtung ausgesendeter Strahlung an einer Grenzfläche zwischen der Gehäusewand, beispielsweise einem Fensterbereich, und dem Dialysat oder dem Blut, wobei die ausgesendete Strahlung unter einem zwischen 0° und 90° liegenden Einfallswinkel auf der Grenzfläche auftrifft,
- Streuung von von der Strahlungsaussendeeinrichtung ausgesendeter Strahlung an einem in dem Dialysat oder in dem Blut enthaltenen Stoff, einschließlich dynamischer Streuung von monochromatischer Laserstrahlung,
- Wechselwirkung von von der Strahlungsaussendeeinrichtung ausgesendeter Strahlung mit einer Identifizierungseinrichtung, die an der Dialysatorvorrichtung oder an der Membranfiltereinrichtung angebracht ist, wie etwa einem Barcode, einem RFID-Code oder einem Farbencodefeld, die ein für die Identifizierung der Dialysatorvorrichtung, einer Komponente derselben, eines in der Dialysatorvorrichtung befindlichen oder durch diese strömenden Mediums, oder der Membranfiltereinrichtung charakteristisches Identifizierungsmerkmal aufweist. Hierbei können alle möglichen Codes wie etwa optische, akustische, elektrische, magnetische und sonstige Codes, auch eine mechanische Form der Codierung, z.B. eine spezielle Gehäuseform, welche gleichzeitig ein optisches Element ist, oder eine Texterkennung zum Einsatz kommen,
- RFID.

Die vielseitige Auswahlmöglichkeit der optischen Wechselwirkung, die die gemessene Strahlung im Innenvolumenbereich mit den dort vorhandenen Elementen untergeht, ermöglicht, dass unterschiedliche Informationen bzw. Arten von Informationen bzw. verschiedenartige Parameter zur Identifizierung des Dialysators, oder zur Erfassung der im Innenvolumenbereich vorhandenen Elemente oder der Membranfiltereinrichtung gemessen werden können.

Die Dialysatorvorrichtung oder die Komponente, z.B. das Gehäuse, ein Deckel oder die Membranfiltereinrichtung, umfassen bei einem, mehreren oder allen Ausführungsbeispielen eine sogenannte passive Identifizierungseinrichtung, die ein für die Identifizierung der Dialysatorvorrichtung oder der Komponente, z.B. der Membranfiltereinrichtung charakteristisches Identifizierungsmerkmal aufweist, das z.B. durch Anstrahlen der Identifizierungseinrichtung mit einer vorbestimmten elektromagnetischen Strahlung auslesbar ist. Dabei kann die Sensorvorrichtung eine Strahlungsaussendeeinrichtung umfassen, die dazu ausgebildet ist, die vorbestimmte elektromagnetische Strahlung, insbesondere zu der Identifizierungseinrichtung, auszusenden. Dabei kann die Signalempfangseinrichtung eine Identifizierungsleseeinrichtung umfassen, die dazu ausgebildet ist, einen für das Identifizierungsmerkmal indikativen Parameter der Strahlung zu detektieren und das Identifizierungsmerkmal zu bestimmen. In dieser Ausführungsform kann die Identifizierungseinrichtung etwa ein Barcode, ein Farbencodefeld oder ein passiver RFID Chip sein. Die Identifizierungseinrichtung ermöglicht, dass Information bezüglich der Identifizierung der Dialysatorvorrichtung oder der Membranfiltereinrichtung besonders zuverlässig, direkt und im Wesentlichen fehler- und störungsfrei gemessen werden kann.

Alternativ dazu kann die Dialysatorvorrichtung oder die Membranfiltereinrichtung eine sogenannte aktive Identifizierungseinrichtung umfassen, die elektromagnetische Strahlung aussenden kann, die ein für die Identifizierung der Dialysatorvorrichtung oder der Membranfiltereinrichtung charakteristisches Identifizierungsmerkmal aufweist. Dabei kann die Signalempfangseinrichtung eine Identifizierungsleseeinrichtung umfassen, die dazu ausgebildet ist, die von der Identifizierungseinrichtung ausgesendete elektromagnetische Strahlung zu detektieren und, etwa nach einer Demodulationsauswertung, das Identifizierungsmerkmal zu bestimmen. Dabei kann die Identifizierungseinrichtung etwa ein aktiver RFID Chip sein. Auch in dieser Ausgestaltung mit einer aktiven Identifizierungseinrichtung kann Information bezüglich der Identifizierung der Dialysatorvorrichtung oder der Membranfiltereinrichtung besonders zuverlässig, direkt und im Wesentlichen fehler- und störungsfrei gemessen werden.

Die Strahlungsaussendeeinrichtung kann mindestens eines oder mehrere der folgenden Merkmale umfassen: eine Lichtquelle, die Licht in einem schmalbandigen Spektralbereich aussendet, wie etwa ein Laser oder eine LED, eine Lichtleitfaser, die aus einer Licht in einem schmalbandigen Spektralbereich aussendenden Lichtquelle ausgesendetes Licht führt und einen Auskopplungsabschnitt aufweist, aus dem das Licht austritt, eine Lichtquelle, die Licht in einem breitbandigen Spektralbereich aussendet, wie etwa eine Halogenlampe, oder eine Lichtleitfaser, die aus einer Licht in einem breitbandigen Spektralbereich aussendenden Lichtquelle ausgesendetes Licht führt und einen Auskopplungsabschnitt aufweist, aus dem das Licht austritt. Diese vielseitige Ausgestaltbarkeit der Strahlungsaussendeeinrichtung ermöglicht, dass verschiedenartige optische Wechselwirkung der in den Innenvolumenbereich ausgesendeten Strahlung mit den dort vorhandenen Elementen induziert und im Ergebnis verschiedenartige Parameter bzw. Informationen bezüglich des Zustands der Dialysatorvorrichtung bzw. der Membranfiltereinrichtung gemessen werden können.

Die Strahlungsaussendeeinrichtung kann eine 1-dimensionale Anordnung oder eine 2-dimensionale Anordnung von mehreren Strahlungsaustrittsbereichen umfassen. Die Strahlungsaussendeeinrichtung kann einen oder mehrere Sender oder Emitter und optional auch Lichtleiter aufweisen. Bei einem oder mehreren Ausführungsbeispielen kann das Gehäuse selbst als Lichtleiter dienen. Es kann eine ausgezeichnete, also eine bevorzugte z.B. lineare Ausrichtung der 1-dimensionalen oder der 2-dimensionalen Anordnung im Betrieb und im verbundenen Zustand der Sensorvorrichtung und der Dialysatorvorrichtung im Wesentlichen parallel zu einer Strömungsrichtung des Dialysats in dem Dialysatbereich, oder parallel zu der Strömungsrichtung des Bluts in dem Blutbereich, oder parallel zu einer Längsachse der Dialysatorvorrichtung, oder entlang des Umfangs des Gehäuses, oder quer oder schräg zu diesen Ausrichtungen, oder kombiniert in zwei oder mehreren dieser Ausrichtungen, also zwei- oder dreidimensional verteilt, ausgerichtet sein. Die Ausgestaltung der Strahlungsaussendeeinrichtung mit einer 1- oder 2-dimensionalen Anordnung mehrerer Strahlungsaustrittsbereiche ermöglicht, dass die ausgesendete Strahlung räumlich verteilt und räumlich selektiv verteilt in den Innenvolumenbereich der Dialysatorvorrichtung eingestrahlt werden und infolgedessen z.B. bei einer geeigneten Ausbildung der Strahlungsmesseinrichtung die gewünschte Information räumlich selektiv gemessen werden kann.

Die Sensorvorrichtung kann eine Strahlungsmesseinrichtung, z.B. mit einer Strahlungsbeeinflussungseinrichtung wie etwa einer Messoptikeinrichtung, und einen Strahlungs-, z.B. Lichtdetektor, der eine strahlungs- oder lichtsensitive Detektorfläche aufweist, umfassen. Die Funktionalität der Strahlungsbeeinflussungseinrichtung kann auch durch das Gehäuse selbst bereitgestellt werden. Das Gehäuse kann z.B. als Lichtleiter ausgebildet oder mit einem oder mehreren Lichtleitern ausgestattet sein.

Die Strahlungsbeeinflussungseinrichtung oder Messoptikeinrichtung kann optional eine Fokaltiefe und / oder eine Fokalrichtung aufweisen. Die Strahlungsbeeinflussungseinrichtung kann auch ausgebildet sein, um Strahlung oder Licht der Lichtquelle an einen bestimmten Punkt im Dialysator abzubilden oder dorthin zu fokussieren. Alternativ oder zusätzlich kann die Strahlungsbeeinflussungseinrichtung dazu dienen, Licht aus dem Dialysator auf die Sensorvorrichtung (Detektor) zu fokussieren und nicht nur abzubilden. Dabei kann die Strahlungsbeeinflussungseinrichtung wie etwa die Messoptikeinrichtung dazu ausgebildet sein, einen Raumbereich aus dem Innenvolumenbereich des Gehäuses der Dialysatorvorrichtung auf die Detektorfläche des Lichtdetektors abzubilden, wobei der abgebildete Raumbereich durch die Fokaltiefe und die Fokalrichtung definiert ist. Die Strahlungsbeeinflussungseinrichtung kann so ausgebildet sein, dass ihre Fokaltiefe und Fokalrichtung so wählbar ist, dass der abgebildete Raumbereich im Bereich des Innenvolumenbereichs wählbar ist. Durch diese Ausbildung der Strahlungsmesseinrichtung kann die gewünschte Information an dem Außen- oder Randbereich oder in manchen Fällen auch aus oder in dem Innenvolumenbereich z.B. zur Identifizierung des Dialysators räumlich selektiv gemessen werden.

Die Sensorvorrichtung kann eine Strahlungsmesseinrichtung mit mindestens einem Strahlungseintrittsbereich und mindestens einem Strahlungs-, z.B. Lichtdetektor umfassen, der dazu ausgebildet ist, Strahlung, wie etwa Licht, die in den mindestens einen Strahlungseintrittsbereich eingetreten ist, zu detektieren und der beispielsweise aus einer Gruppe ausgewählt ist, die folgendes umfassen kann: eine Photodiode, einen Phototransistor, einen CMOS-Lichtdetektor, einen Photomultiplier mit einem oder mehreren linienförmig eindimensional oder flächig zweidimensional verteilt angeordneten Multiplierelementen, eine Avalanche-Photodiode mit einem oder mehreren linienförmig eindimensional oder flächig zweidimensional verteilt angeordneten Photodiodenelementen, einen 1-dimensionalen oder 2-dimensionalen CCD-Sensor, und eine 1-dimensionale Anordnung oder eine 2-dimensionale Anordnung einer Vielzahl von Photodioden, Phototransistoren, CMOS-Lichtdetektoren, Photomultiplier oder Avalanche-Photodioden, oder andere Detektoren wie etwa MPPC (Multi Photon Pixel Counter, oder sonstige Sensorelemente. Die vielseitige Wählbarkeit der Art des Strahlungsdetektors ermöglicht es, für die Messung verschiedenartiger Parameter bzw. Informationen jeweils optimal geeignete Strahlungsdetektoren bereitzustellen.

Ein jeweiliger Strahlungseintrittsbereich kann einen Einkoppelbereich eines optischen Lichtleiters oder einer Lichtleitfaser umfassen, der/die dazu ausgebildet ist, die Strahlung zu einem dem Strahlungseintrittsbereich zugeordneten Lichtdetektor zu leiten. Diese Ausgestaltung ermöglicht es, den Strahlungseintrittsbereich und den diesem zugeordneten Lichtdetektor räumlich voneinander beabstandet einzurichten bzw. räumlich zu entkoppeln. So können ggf. auch Lichtdetektoren zum Einsatz kommen, die räumlich relativ viel Platz einnehmen, um Strahlung zu messen, die dicht an der Dialysatorvorrichtung an dem Strahlungseintrittsbereich "aufgefangen" wird.

Die Sensorvorrichtung kann eine Strahlungsmesseinrichtung mit mindestens einem Lichtdetektor und einer Wellenlängenauswahleinrichtung, wie etwa einem Monochromator, umfassen, wobei die Wellenlängenauswahleinrichtung dazu ausgebildet ist, einen schmalbandigen Wellenlängenbereich, der eine Nachweiswellenlänge umfasst, aus einem beispielsweise breitbandigeren Wellenlängenbereich der von der Strahlungsaussendeeinrichtung ausgesendeten Strahlung, z.B. Licht, so auszuwählen, dass eine optische Wechselwirkung, wie etwa Absorption oder Anregung von Lumineszenz- oder Fluoreszenzemission, mit einem ausgewählten Stoff, der sich im Betrieb in dem Innenvolumenbereich des Gehäuses befindet, erfolgt. Dabei kann der Stoff etwa ein Bestandteil des Dialysats und/oder des Bluts sein. Die in dieser Ausgestaltung ermöglichte wellenlängenselektive Messung von Strahlung, z.B. Licht, ermöglicht auch eine stoffspezifische Messung.

Die Sensorvorrichtung kann dazu ausgelegt sein, mindestens einen oder mehrere der folgenden Parameter oder Größen zu ermitteln:
Messen eines für die Konzentration eines urämischen Toxins indikativen Parameters im Blutbereich des Innenvolumenbereichs, wie etwa die Absorption von Licht mit einer Messwellenlänge, das von dem urämischen Toxin absorbiert wird;
Messen eines für die Konzentration eines urämischen Toxins indikativen Parameters im Dialysatbereich des Innenvolumenbereichs, wie etwa die Absorption von Licht mit einer Messwellenlänge, das von dem urämischen Toxin absorbiert wird; und
Messen eines für eine physikalische Eigenschaft des Bluts, wie etwa Viskosität oder Hämatokrit-Konzentration, indikativen Parameters im Blutbereich.

Die Möglichkeit der Ausbildung der Sensorvorrichtung für die genannten, unterschiedlichen Aufgaben ermöglicht, dass in der Praxis häufig verwendete und/oder den Fortgang der Dialysetherapie am Patienten kennzeichnende Parameter gemessen werden können. Entsprechend können auf der Grundlage der Messung dieser Parameter Einstellungen bzw. Betriebsparameter einer Dialysemaschine im Verlauf der Dialysetherapie an den Fortgang der Dialysetherapie angepasst gesteuert werden.

Das Gehäuse der Dialysatorvorrichtung gemäß dem dritten Aspekt der Erfindung kann so ausgebildet sein, dass die Sensorvorrichtung in einer oder mehreren vorbestimmten Positionen relativ zu dem Gehäuse formschlüssig lösbar fixiert werden kann. In einer vorteilhaften Ausgestaltung kann das Gehäuse an seiner Außenseite an einer oder mehreren Positionen erste Koppelbereiche aufweisen, und die Sensorvorrichtung kann einen zweiten Koppelbereich aufweisen, der formschlüssig mit dem ersten Koppelbereich eingreifbar, z.B. andrückbar ausgebildet ist. Das Anbringen der Sensorvorrichtung an einer vorbestimmten Position relativ zu dem Gehäuse ermöglicht es, mit einer Sensorvorrichtung die gewünschte Information an unterschiedlichen Dialysatorvorrichtungen zuverlässig und unter vergleichbaren Messbedingungen und damit auch vergleichbar zu messen

Vorzugsweise ist die Sensorvorrichtung gemäß dem zweiten Aspekt der Erfindung wieder verwendbar ausgebildet. Durch die Wiederverwendbarkeit wird es effizient bzw. kostengünstig, auch relativ aufwändige, komplexe und entsprechend in der Anschaffung teure Sensorik einzusetzen.

Die an oder mit dem Gehäuse der Dialysatorvorrichtung lösbar, insbesondere formschlüssig lösbar, befestigbare Ausbildung der Sensorvorrichtung kann z.B. durch mindestens einen der folgenden Mechanismen bewirkt werden:
die Sensorvorrichtung ist als eine Clipeinrichtung mit mindestens einem oder mehreren flexiblen Clipmanschetten ausgebildet, wobei die Clipmanschette dazu ausgebildet ist, das Gehäuse in einem Winkelbereich von mehr als 180°, vorzugsweise mehr als 270° und noch mehr bevorzugt mehr als 320° zu umgreifen,
die Sensorvorrichtung umfasst einen Grundkörper und eine Clipeinrichtung mit mindestens einem Arm oder z.B. zwei oder mehreren Armen wie etwa ein Paar von an dem Grundkörper befestigten elastisch-flexiblen Cliparmen, oder einem, zwei oder mehreren an dem Grundkörper elastisch-flexibel angebrachten, z.B. angelenkten, Cliparmen, der/die dazu ausgebildet ist/sind, zwischen sich das Gehäuse aufzunehmen und aufgrund ihrer elastisch-flexiblen Ausgestaltung oder Anlenkung das Gehäuse clipartig zu umklammern,
die Sensorvorrichtung umfasst ein erstes Glied einer Verbindungseinrichtung, wie etwa eine Schwalbenschwanzeinrichtung, eine Schraube bzw. ein Klettband, und die Dialysatorvorrichtung ein zweites Glied der Verbindungseinrichtung, wie etwa eine Schwalbenschwanzaufnahmeeinrichtung, ein zum Einschrauben der Schraube ausgebildetes Loch oder Schraubengewinde bzw. ein Gegenstück zu dem Klettband, wobei das erste Glied in das zweite Glied oder das zweite Glied in das erste Glied lösbar formschlüssig eingreifbar ausgebildet ist, und wobei die beiden Teile des Klettbandes bzw. der anderen Verbindungsvorrichtungen auch beide auf dem "Clip" angebracht sein können, der dann durch entsprechendes enges Anziehen der Verbindungseinrichtung die Dialysatorvorrichtung fixiert,
die Sensorvorrichtung wird mittels einer elastischen Spanneinrichtung, wie etwa einem Gummiring, an dem Gehäuse festgespannt, wobei die Spanneinrichtung dazu ausgebildet ist, in einem elastisch gespannten Zustand die Sensorvorrichtung und das Gehäuse der Dialysatorvorrichtung zu umzuspannen,
die Sensorvorrichtung umfasst einen Grundkörper und mindestens ein Paar von an dem Grundkörper angelenkten oder mit dem Grundkörper elastisch-flexibel verbundenen Armen bzw. Manschetten, die jeweils einen distalen Endbereich mit einem dort vorgesehenen ersten Glied einer Haken- oder Eingreifeinrichtung aufweisen, wobei die Arme bzw. Manschetten dazu ausgebildet sind, das Gehäuse in einem Winkelbereich von mehr als 180°, vorzugsweise mehr als 270°, und mehr bevorzugt mehr als 320°, umgreifen und eine elastisch spannbare Spanneinrichtung mit zwei gegenüberliegenden Endabschnitten, an denen jeweils eine zum lösbaren Eingreifen mit einem ersten Glied der Haken- oder Eingreifeinrichtung ausgebildetes zweites Glied einer Haken- oder Eingreifeinrichtung vorgesehen ist, wobei in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung eingreifen kann, und
die Sensorvorrichtung ist dazu ausgebildet, beispielsweise in einer Vertiefung, in der Gehäusewand der Dialysatorvorrichtung lösbar integriert zu werden.

Das Gehäuse der Dialysatorvorrichtung kann eine Gehäusewand mit mindestens einem für ein Signal durchlässigen Fensterbereich umfassen. Die Ausbildung mit einem für das Signal besonders durchlässigen Fensterbereich ermöglicht, dass auch an einem Gehäuse mit einem ansonsten für das Signal weniger durchlässigen Material der Gehäusewand, die jedoch beispielsweise aus einem kostengünstigen Material hergestellt sein kann, genaue Messungen der gewünschten Information bzw. Parameter mit der an dem bzw. in der Nähe des Fensterbereichs anzubringenden Sensorvorrichtung durchgeführt werden können.

Die Gehäusewand kann im Wesentlichen zylinderförmig ausgebildet sein. Diese Ausgestaltung ermöglicht, dass die Sensorvorrichtung leicht in entlang einer Längsrichtung der Gehäusewand verschiedenen Positionen und mit in Bezug auf die Längsachse azimutal unterschiedlichen Blickrichtungen angebracht werden kann.

In einer ersten Ausgestaltung kann die Membranfiltereinrichtung in das Gehäuse der Dialysatorvorrichtung herausnehmbar eingebracht werden. Diese Ausgestaltung ermöglicht, dass nur die Membranfiltereinrichtung als Einmalartikel ausgebildet sein kann, während das Gehäuse der Dialysatorvorrichtung, insbesondere z.B. wenn darin relativ teure Fensterbereiche integriert sind, wieder verwendbar sein kann.

In einer alternativen, zweiten Ausgestaltung kann die Dialysatorvorrichtung zusätzlich noch ein Innengehäuse umfassen, das in dem Gehäuse lösbar fixiert werden kann und das die Membranfiltereinrichtung im Wesentlichen umschließt. Dabei kann das Innengehäuse, insbesondere mit der darin angeordneten Membranfiltereinrichtung, in das Gehäuse der Dialysatorvorrichtung herausnehmbar eingebracht werden. Diese Ausgestaltung ermöglicht, dass das Innengehäuse mit der Membranfiltereinrichtung als Einmalartikel ausgebildet sein kann, während das Gehäuse der Dialysatorvorrichtung, insbesondere z.B. wenn darin relativ teure Fensterbereiche integriert sind, wieder verwendbar sein kann.

In der ersten Ausgestaltung der Dialysatorvorrichtung kann zumindest die Membranfiltereinrichtung als Einmalartikel ausgebildet sein. In der zweiten Ausgestaltung können das Innengehäuse und die Membranfiltereinrichtung als Einmalartikel ausgebildet sein. Bei dieser Ausführung kann bei einem oder mehreren Ausführungsbeispielen die Sensorik auch direkt in das Dialysatorgehäuse eingebracht sein, wobei nur die elektrischen Schnittstellen außen am Dialysator kontaktiert werden.

In den vorgenannten ersten und zweiten Ausgestaltungen ist es bevorzugt, dass das Gehäuse so ausgebildet ist, dass es leicht und gründlich gereinigt werden kann.

Die Dialysatorvorrichtung kann auch als Ganzes als Einmalartikel ausgebildet sein. In dieser Ausgestaltung ist ein Reinigen der Dialysatorvorrichtung oder von Komponenten derselben nach einer an einem Patienten durchgeführten Dialysetherapie nicht erforderlich.

Der Innenvolumenbereich der Dialysatorvorrichtung kann einen im Betrieb von einem Dialysat durchströmbaren Dialysatbereich und einen im Betrieb von Blut durchströmbaren Blutbereich umfassen, wobei der Dialysatbereich von dem Blutbereich durch die Membranfiltereinrichtung räumlich getrennt sind. Die Membranfiltereinrichtung kann dazu ausgebildet sein, im Betrieb einen durch Konzentrationsgradienten von in dem Blut bzw. in dem Dialysat gelösten Stoffen getriebenen Stoffaustausch zwischen dem Blut und dem Dialysat zu ermöglichen. Diese Ausgestaltungen ermöglichen eine Verwendung der Dialysatorvorrichtung gemäß dem dritten Aspekt der Erfindung in herkömmlichen Dialysemaschinen.

Die Dialysatorvorrichtung oder eine Dialysemaschine gemäß dem vierten Aspekt der Erfindung kann zumindest einen Gehäusehalter umfassen, der dazu ausgebildet ist, im Betrieb mit der Dialysemaschine zumindest mechanisch verbunden zu sein und die Dialysatorvorrichtung an der Dialysemaschine lösbar zu fixieren. Der Gehäusehalter kann die Dialysatorvorrichtung, ggf. im Betrieb zusammen mit der an der Dialysatorvorrichtung angebrachten Sensorvorrichtung, zuverlässig halten.

In der Dialysemaschine kann die Sensorvorrichtung in dem Gehäusehalter integriert sein. Diese Ausgestaltung vereinfacht die Handhabung der Sensorvorrichtung, weil diese nicht in einem zusätzlichen Arbeitsschritt an dem Gehäuse der Dialysatorvorrichtung angebracht zu werden braucht.

Die Erfindung wird im Folgenden anhand von in den beigefügten Figuren gezeigten Ausführungsformen der erfindungsgemäßen Vorrichtung sowie von Sensorvorrichtungen und der Dialysatorvorrichtungen näher beschrieben. Es zeigen:
Figur 1 eine schematische Darstellung eines Blutkreislaufes und eines beispielsweise in einer Dialysemaschine angeordneten Dialysatkreislaufes in einem Dialysebetrieb, wobei an den Blutkreislauf als extrakorporaler Blutkreislauf ein Patient angeschlossen ist;

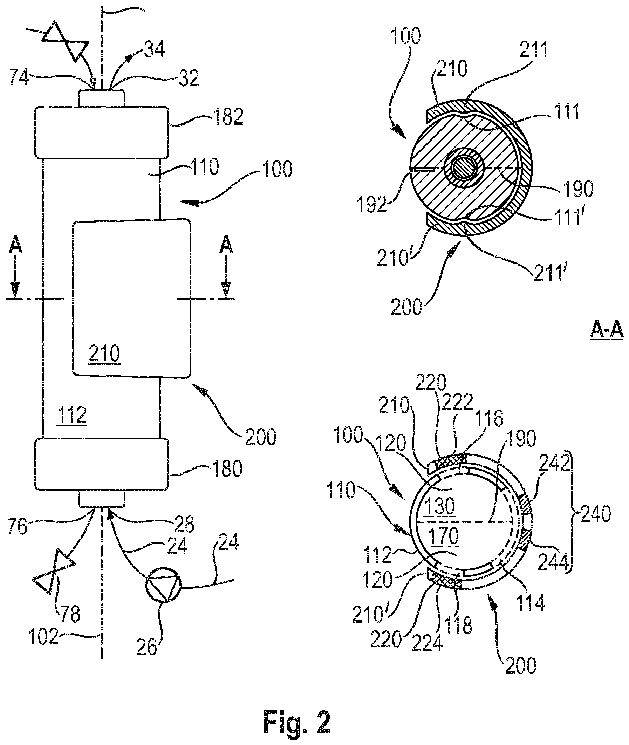

Figur 2 im linken Bereich eine schematische Seitenansicht eines System mit einer ersten Ausführungsform einer Sensorvorrichtung, die an einer ersten Ausführungsform einer Dialysatorvorrichtung lösbar operativ verbunden ist, im mittleren Bereich eine schematische Draufsicht auf das System mit der Sensorvorrichtung und der Dialysatorvorrichtung aus dem linken Bereich, und im rechten Bereich einen schematischen Querschnitt entlang der Linie A-A durch das System mit der Sensorvorrichtung und der Dialysatorvorrichtung aus dem linken Bereich;

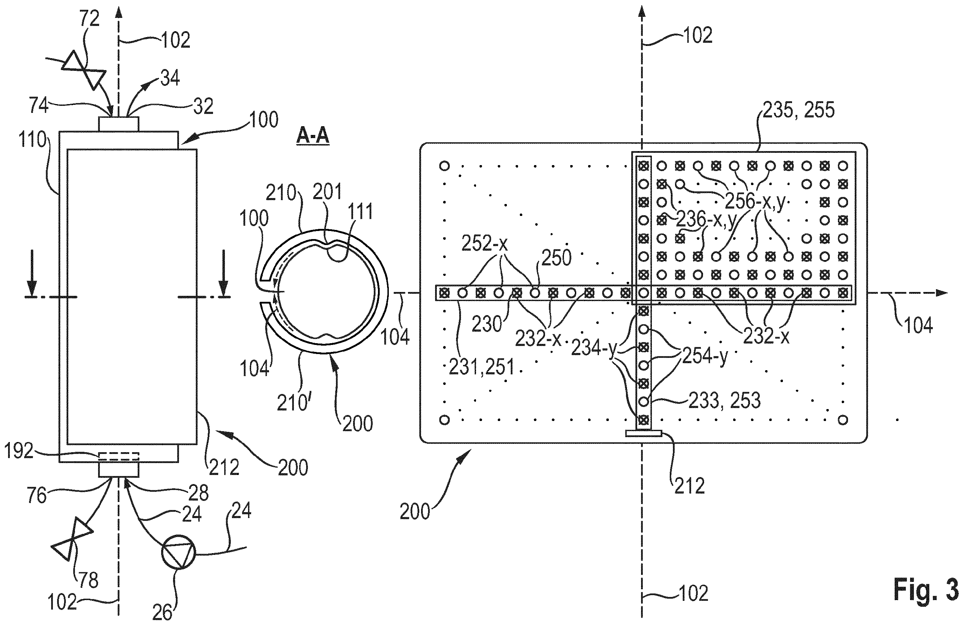

Figur 3 im linken Bereich eine schematische Seitenansicht eines System mit einer zweiten Ausführungsform einer Sensorvorrichtung, die an einer zweiten Ausführungsform einer Dialysatorvorrichtung lösbar operativ verbunden ist, im mittleren Bereich einen schematischen Querschnitt entlang der Linie A-A durch das System mit der Sensorvorrichtung und der Dialysatorvorrichtung aus dem linken Bereich, und im rechten Bereich eine schematische Darstellung der Sensorvorrichtung aus dem linken Bereich in einem auseinander gerollten Zustand; und

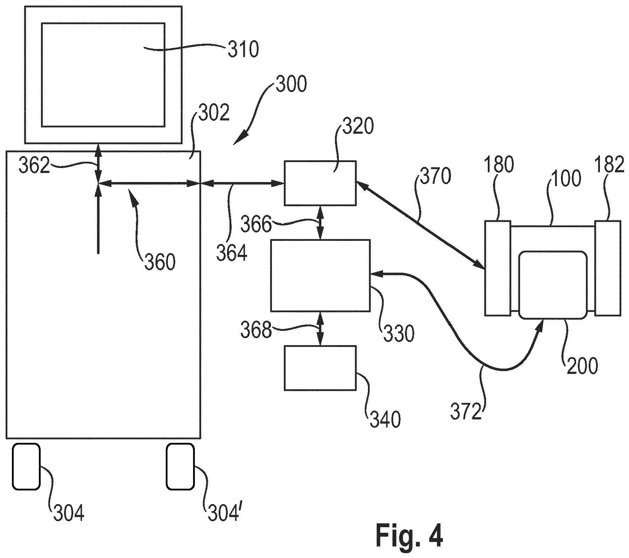

Figur 4 eine Blockdarstellung einer Dialysemaschine und ihrer funkionellen Komponenten einschließlich einer Ausführungsform eines Systems mit einer Ausführungsform einer Sensorvorrichtung, die mit einer Ausführungsform einer Dialysatorvorrichtung lösbar verbunden ist.

### Ausführungsformen der Erfindung

Fig. 1 zeigt schematisch eine Dialysemaschine 300 zum Durchführen einer Hämodialysebehandlung eines Patienten 10. Wie eingangs bereits erwähnt, umfasst die Dialysemaschine 300 den Dialysatkreislauf 40, den in Bezug auf den Patienten 10 extrakorporal geführten Blutkreislauf 20 und einen Dialysator, d.h. die Dialysatorvorrichtung 100 mit dem Innenvolumenbereich 120 und der darin angeordneten Membranfiltereinrichtung 190 mit der Membran, an der der Stoffaustausch zwischen dem im Blutkreislauf 20 geführten Blut des Patienten 10 und dem im Dialysatkreislauf 40 geführten Dialysat stattfindet.

Der Dialysatkreislauf 40 kann im Wesentlichen innerhalb der Dialysemaschine 300 angeordnet sein, aber auch anders ausgestaltet sein, und umfasst im Einzelnen die Komponenten 42, 44, 54, 60 zur Dialysataufbereitung, den Dialysatbereich 170 der Dialysatorvorrichtung 100, ein Dialysatoreingangsventil 72 und einen Dialysateinlass 74 zum Einlassen des aufbereiteten Dialysats in den Dialysatbereich 170, einen Dialysatauslass 76 und ein Dialysatorausgangsventil 78 zum Auslassen des gebrauchten Dialysats aus dem Dialysatbereich 170 und zur Abfuhr des Dialysats zu den Elementen 84, 80 und 86 der Dialysatabfuhr. Die Komponenten der Dialysataufbereitung umfassen typischerweise eine Wasseraufbereitungseinrichtung 42 zum Bereitstellen eines Stromes von aufbereitetem Wasser in die Dialysatzufuhrleitungen 62, 62', ein in einem Reservoir 44 bereitgestelltes Bicarbonat-Konzentrat, das mittels einer steuerbaren Bicarbonat-Pumpe 46 in dosierbarer Menge durch den Bicarbonat-Zufluss 48 in die Dialysatorzufuhrleitung 62 eindosiert wird, ein in einem Reservoir 54 bereitgestelltes Säure-Konzentrat, das mittels einer dosierbaren Säure-Pumpe 56 in dosierbarer Menge durch den Säure-Zufluss 58 in die Dialysatorzufuhrleitung 62 eindosiert werden kann, und eine stromabwärts der Zuflüsse 48, 58 in der Dialysatzufuhrleitung 62 in-line angeordnete Bilanzierungseinrichtung 60. Die Bilanzierungseinrichtung 60 oder eine in ihr enthaltene oder mit ihr verbundene Steuereinrichtung ist dazu ausgebildet, die Zusammensetzung und die abgegebene Menge, d.h. den Dialysatstrom, in einer auf den Patienten 10 abgestimmten und für den Fortgang der Dialysebehandlung spezifischen Weise zu steuern, und insbesondere dazu, die für diese Steuerung erforderlichen Steuersignale für die Bicarbonat-Pumpe 46, die Säure-Pumpe 56, eine stromabwärts der Bilanzierungseinrichtung 60 in der Dialysatzufuhrleitung 62' angeordnete Flusspumpe 64 für den Dialysatzugang und eine stromabwärts des Dialysatbereichs 170 in der Dialysatausflussleitung 82, 82' angeordnete Flusspumpe 84 zu erzeugen und die Flusspumpe 64 für den Dialysatzugang und die Flusspumpe 84 für die Dialysatabfuhr zu steuern.

Das von der Flusspumpe 64 dosierte und stromauf derselben aufbereitete Dialysat wird durch das Dialysatoreingangsventil 72 und den Dialysateinlass 74 in den Dialysatbereich 170 der Dialysatorvorrichtung 100 geführt. Nach Durchführung des Stoffaustauschs mit dem Blut des Patienten 10 durch die Membran der Membranfiltereinrichtung 190 strömt das Dialysat weiter durch den Dialysatauslass 76 und wird durch das Dialysatorausgangsventil 78 dem Dialysatausfluss- bzw. abfuhrsystem 84, 80, 86 zugeführt. Das Dialysatabfuhrsystem umfasst eine typischerweise stromabwärts der Dialysatausfluss-Flusspumpe 84 in der Dialysatausflussleitung 82, 82' angeordnete Bilanzierungseinrichtung 80 und eine stromabwärts der Bilanzierungseinrichtung 80 in der Dialysatausflussleitung 82' angeordnete Dialysatabfuhr 86. Die Dialysatabfuhr 86 kann z.B. als ein ausleerbarer oder abführbarer bzw. entsorgbarer Auffangbehälter ausgebildet sein. In vielen Fällen kann das verbrauchte Dialysat aber auch direkt in den Abfluss und damit in die Wiederaufbereitung oder Kanalisation laufen. Der Dialysatkreislauf 40 umfasst ferner eine Bypass-Leitung 68 zu dem Dialysatbereich 170 der Dialysatorvorrichtung 100. Die Bypass-Leitung 68 kann mittels eines Bypass-Ventils 66 geöffnet bzw. geschlossen werden und ermöglicht einen Bypass von Dialysat bezüglich des Dialysatbereichs 170 vom Dialysatzugang 64 direkt zum Dialysatausfluss 84.

Im Einzelnen umfasst der extrakorporale Blutkreislauf 20 ein arterielles Schlauchsystem 24, einen Blutbereich 130 der Dialysatorvorrichtung 100 und ein venöses Schlauchsystem 34. Der extrakorporale Blutkreislauf 20 wird in einer Hämodialysebehandlung durch medizinisches Fachpersonal eingerichtet und umfasst ein arterielles Schlauchsystem 24 zum Ausführen von zu reinigendem Blut aus dem Patienten 10, den Blutbereich 130 der Dialysatorvorrichtung 100 und ein venöses Schlauchsystem 34 zum Rückführen von gereinigtem Blut zum Patienten 10. Das arterielle Schlauchsystem 24 umfasst einen arteriellen Zugang 22 zum arteriellen Blutgefäßsystem des Patienten 10, der beispielsweise als arterielle Nadel oder Katheter eingerichtet werden kann, und eine arterielle Blutpumpe 26 zum Pumpen von arteriellem Blut des Patienten 10 vom arteriellen Zugang 22 durch den Bluteinlass 28 in den Blutbereich 130 der Dialysatorvorrichtung 100 und weiter durch das venöse Schlauchsystem 34 zu einem venösen Zugang 36 in das venöse Blutgefäßsystem des Patienten 10. Die arterielle Blutpumpe 26 kann z.B. am oder im arteriellen Schlauchsystem 24 eingeschlaucht oder angeordnet sein. Das venöse Schlauchsystem 34 führt das im Dialysatbereich 130 gereinigte bzw. behandelte Blut von dem Blutauslass 32 des Dialysatbereichs 130 zu dem venösen Zugang 36 des Patienten 10 zurück. Dadurch wird der extrakorporale Blutkreislauf 20 geschlossen.

In der in Fig. 1 gezeigten schematischen Darstellung des Dialysatkreislaufs 40 und des Blutkreislaufs 20 findet, wie bereits erwähnt, der Stoffaustausch zwischen dem Dialysat und dem Blut des Patienten 10 im Dialysator bzw. in der Dialysatorvorrichtung 100 gemäß dem dritten Aspekt der Erfindung, genauer an der Membran der darin angeordneten Membranfiltereinrichtung 190 statt. Letztere unterteilt den Innenvolumenbereich 120 eines Gehäuses 110 der Dialysatorvorrichtung 100 in einen Dialysatbereich 170, der von dem über den Dialysatzulass 74 zuströmenden und durch den Dialysatabfluss 76 abgeführten Dialysat durchströmt wird, und den Blutbereich 130, der von dem durch den Bluteinlass 28 zuströmenden, zu reinigenden Blut des Patienten 10 und dem behandelten bzw. gereinigten und durch den Blutauslass 32 ab- und dem Patienten 10 zurückgeführten Blut durchströmt wird. Die Dialysatorvorrichtung 100 und/oder eine daran oder in ihr angeordnete Komponente, z.B. die darin angeordnete Membranfiltereinrichtung 190, umfasst eine passive oder eine aktive Identifizierungseinrichtung 192, die ein für die Identifizierung der Dialysatorvorrichtung 100 und/oder eine Komponente derselben, z.B. die Membranfiltereinrichtung 190, charakteristisches Identifizierungsmerkmal aufweist.

In einer Ausführungsform der erfindungsgemäßen Vorrichtung ist die passive Identifizierungseinrichtung 192 ein passiver RFID (Englisch: radio frequency identification) Chip, bei dem das Identifizierungsmerkmal durch Anstrahlen mit einer vorbestimmten elektromagnetischen Strahlung, deren Frequenz im z.B. RF (Englisch: radio frequency) Bereich liegt, auslesbar ist. In einer anderen Ausführungsform umfasst die passive Identifizierungseinrichtung 192 einen Code wie etwa einen Barcode oder ein Farbencodefeld, bei dem das Identifizierungsmerkmal durch Anstrahlen mit einer im optischen Bereich liegenden elektromagnetischen Strahlung mit einer Wellenlänge im Bereich des infraroten (IR), nahinfraroten, sichtbaren oder ultravioletten (UV) Lichts auslesbar ist.

Die in Fig. 1 gezeigten Dialysatorvorrichtung 100 weist ein Ausführungsbeispiel der erfindungsgemäßen Vorrichtung mit, oder in Form, einer Sensorvorrichtung 200 auf, die mit dem Dialysator lösbar und operativ verbindbar ist. Die Sensorvorrichtung 200 umfasst eine Signalempfangseinrichtung 210, die dazu ausgebildet ist, mindestens ein Signal, wie etwa ein Strahlungssignal oder Ultraschallsignal, von dem Außen- oder Randbereich oder auch aus dem Innenvolumenbereich 120 des Gehäuses 110 der Dialysatorvorrichtung 100 zu empfangen, und kann auch außen am Gehäuse angebracht oder als Bestandteil des Gehäuses ausgebildet sein. Das empfangene Signal ist charakteristisch für den Zustand bzw. die Identifizierung der Dialysatorvorrichtung 100 bzw. der darin angeordneten Membranfilereinrichtung 190. In einer Ausführungsform umfasst die Signalempfangseinrichtung 210 eine Identifizierungsleseeinrichtung 212, die dazu ausgebildet ist, einen für das Identifizierungsmerkmal der Identifizierungseinrichtung 192 indikativen Parameter der Strahlung zu detektieren und das Identifizierungsmerkmal und darüber die Identifizierung der Dialysatorvorrichtung 100 bzw. der Membranfiltereinrichtung 190 zu bestimmen. Damit das Signal aus dem Innenvolumenbereich 120 des Gehäuses 110 zu der außerhalb des Gehäuses 110 angeordneten Signalempfangseinrichtung 210 gelangen kann, ist das Gehäuse 110 für das Signal zumindest bereichsweise, oder als Ganzes, im Wesentlichen durchlässig.

Zum Anstrahlen der passiven Identifizierungseinrichtung 192 umfasst die Sensorvorrichtung 200 eine Strahlungsaussendeeinrichtung 240, die dazu ausgebildet ist, die vorbestimmte Strahlung, z.B. elektromagnetische Strahlung oder Ultraschallstrahlung, zum Bestrahlen zu der passiven Identifizierungseinrichtung 192 auszusenden, um diese dazu zu aktivieren, modifizierte elektromagnetische oder ultraschallakustische Strahlung, die das Identifizierungsmerkmal bzw. einen dafür indikativen Parameter enthält, auszusenden.

In einer Ausführungsform mit einem passiven RFID-Chip als passive Identifizierungseinrichtung 192 umfasst die Strahlungsaussendeeinrichtung 240 eine RF-Strahlungsquelle (nicht gezeigt). In der Ausführungsform, bei der die passive Identifizierungseinrichtung 192 ein Code wie beispielsweise ein Barcode, eine Datamatrix, allgemein ein 2-dimensionaler oder ein 3-dimensionaler Code, z.B. ein farbiger 2D Code, oder ein Farbencodefeld ist, umfasst die Strahlungsaussendeeinrichtung 240 eine z.B. optische oder akustische Strahlungsquelle zum Bestrahlen des Codes, und die Identifizierungseinrichtung 212 der Signalempfangseinrichtung 212 umfasst beispielsweise einen Codescanner zum Auslesen des Codes wie etwa einen Barcodescanner zum Lesen des Barcodes oder eine Kamera, beispielsweise mit einem zweidimensionalen CCD-Sensor oder einem 2D-Codeleser, die dazu ausgebildet sind, die von dem Code, z.B. Barcode bzw. Farbencodefeld reflektierte Strahlung, z.B. optisches Licht zu detektieren, in geeigneter Weise zu analysieren und den in der detektierten Strahlung enthaltenen indikativen Parameter bzw. das Identifizierungsmerkmal zu erkennen.

In einer anderen Ausführungsform umfasst die Dialysatorvorrichtung 100 bzw. die Membranfiltereinrichtung 190 eine aktive Identifizierungseinrichtung 192, wie etwa einen aktiven RFID-Chip, die elektromagnetische Strahlung aussenden kann, die ein für die Identifizierung der Dialysatorvorrichtung 100 oder der Membranfiltereinrichtung 190 charakteristisches Identifizierungsmerkmal aufweist. Dabei ist es nicht erforderlich, in der Sensorvorrichtung 200 eine Strahlungsaussendeeinrichtung zum Anstrahlen der Identifizierungseinrichtung mit einer vorbestimmten elektromagnetischen Strahlung vorzusehen. Die Signalempfangseinrichtung 210 umfasst, wie in den Ausführungsformen mit einer passiven Identifizierungseinrichtung 192, eine Identifizierungsleseeinrichtung 212, die dazu ausgebildet ist, die von der (hier aktiven) Identifizierungseinrichtung 192, z. B. dem aktiven RFID-Chip, ausgesendete elektromagnetische Strahlung zu detektieren und, etwa nach einer Demodulationsauswertung, das Identifizierungsmerkmal zu erkennen.

In einer anderen Ausführungsform umfasst die Signalempfangseinrichtung 210 einen Empfänger für ein elektrisches Signal, das indikativ ist für eine zu messende Kapazität und/oder eine zu messende Induktivität, die charakteristisch für den Zustand bzw. die Identifizierung der Dialysatorvorrichtung 100 und/oder der Membranfiltereinrichtung 190 ist.

In einer anderen Ausführungsform kann die Signalempfangseinrichtung 210 dazu ausgebildet sein, Signale von aus dem Innenvolumenbereich 120 austretenden Ultraschall-Strahlung zu empfangen. Die Ultraschall-Strahlung kann in den Innenvolumenbereich 120 eingestrahlt werden und in dem Dialysatbereich 170 und/oder dem Blutbereich 130 reflektiert werden oder diese durchqueren. In dieser Ausführungsform umfasst die Signalaussendeeinrichtung 240 einen Emitter zum Aussenden von Ultraschall-Strahlung auf das Gehäuse oder in den Innenvolumenbereich 120, etwa fokussiert in den Dialysatbereich 170 oder in den Blutbereich 130 oder in den Bereich der Membran der Membranfiltereinrichtung 190. In dem Innenvolumenbereich 120 von den dort in Flüssigkeit (Dialysat und/oder Blut) gelösten Stoffen und/oder von der Membran der Membranfiltereinrichtung 190 beeinflusste eingestrahlte Ultraschall-Strahlung kann in einer Reflexionsanordnung, in einer Transmissionsanordnung oder in einer Streuungsanordnung der für die Detektion der Ultraschall-Strahlung ausgelegten Signalempfangseinrichtung 210 in Bezug auf die Ultraschall-Strahlung aussendende Signalaussendeeinrichtung 240 angeordnet sein.

In einer Reflexionsanordnung ist die Richtung der zu detektierenden Strahlung, hier z.B. Ultraschall-Strahlung, im Wesentlichen entgegengesetzt, z.B. in einem Winkel von 180° oder nahezu 180°, in Bezug auf die Richtung der eingestrahlten Strahlung gerichtet. Die Reflektion kann auch unter einem anderen Winkel als 180 Grad zur Richtung der eingestrahlten Strahlung erfolgen, also z.B. in einer schrägen Richtung oder rechtwinkligen Richtung. In einer Transmissionsanordnung ist die Richtung der zu detektierenden Strahlung im Wesentlichen parallel, d.h. in einem Winkel von 0° oder nahezu 0°, zu der Richtung der eingestrahlten Strahlung gerichtet. Bei einer Streuungsanordnung ist die Richtung der zu detektierenden Strahlung unter einem von 0° und 180° im Wesentlichen unterschiedlichen Winkel, z.B. 90°, gerichtet. Die hier für Ultraschall-Strahlung eingeführte Terminologie (Reflexions-, Transmissions- und Streuungsanordnung) von zu messender Strahlung in Bezug auf die eingestrahlte Strahlung wird hierin auch für optische elektromagnetische Strahlung verwendet.

Wenn die Gehäusewand 110 für optische elektromagnetische Strahlung, d.h. hierin für infrarote (IR), nahinfrarote, sichtbare oder ultraviolette (UV) Lichtstrahlung zumindest bereichsweise durchlässig ist, dann können mit der Sensorvorrichtung 200 gemäß dem dargestellten Ausführungsbeispiel der Erfindung auch verschiedenartige optische Messungen direkt an der Dialysatorvorrichtung 100 ausgeführt werden, aus denen Information zur Identifizierung des Dialysators und/oder bezüglich des Zustands oder einer Eigenschaft, z. B. einer Stoffkonzentration im Dialysat oder Blut oder einer daraus abgeleiteten Größe, oder der Geschwindigkeit in den Fasern der Dialysatorvorrichtung, oder der Braunschen Bewegung, oder der Dialysance der Dialysatorvorrichtung 100 oder der Membranfiltereinrichtung 190 abgeleitet werden können. Derartige optische Messungen können in Reflexions-, Transmissions- und Streu-Anordnungen und ferner auch in Brechungsanordnung einer für die Messung einer Lichtintensität ausgebildeten Signalempfangseinrichtung 210 in Bezug auf eine optische Strahlungsaussendeeinrichtung 240 durchgeführt werden. In einer Ausführungsform umfasst die Signalempfangseinrichtung 210 eine optische Strahlungsmesseinrichtung 220, die einen ersten optischen Strahlungsdetektor 222 und einen zweiten optischen Strahlungsdetektor 224 umfasst (siehe Fig. 2).

Die Sensorvorrichtung 200 kann bei dem dargestellten Ausführungsbeispiel eine Strahlungsaussendeeinrichtung 240 umfassen, die dazu ausgebildet ist, Strahlung, wie etwa optische Strahlung (d.h. Licht) oder Ultraschall-Strahlung, in den Innenvolumenbereich 120 des Gehäuses 110 der Dialysatorvorrichtung 100 auszusenden. Entsprechend kann die Signalempfangseinrichtung 210 dazu ausgebildet sein, die Intensität von aus dem Innenvolumenbereich 120 austretender elektromagnetischer Strahlung oder Ultraschall-Strahlung, die von der Strahlungsaussendeeinrichtung 240 in den Innenvolumenbereich 120 eingestrahlt worden ist, zu messen. Die von der Signalempfangseinrichtung 210 gemessene Strahlung kann das Ergebnis einer für den Zustand der Dialysatorvorrichtung 100 charakteristischen Wechselwirkung der eingestrahlten Strahlung sein, die im Innenvolumenbereich 120 des Gehäuses 110 zwischen der Strahlung und einem oder mehreren der folgenden Objekte stattfindet: dem Dialysat im Dialysatbereich 170, dem Blut im Blutbereich 130, einem in dem Dialysat und/oder in dem Blut enthaltenen Stoff, der Membranfiltereinrichtung 190, und einem in bzw. an der Membranfiltereinrichtung 190 festgehaltenen Stoff, der beispielsweise aus dem Blut bzw. dem Dialysat entstammt. Dabei wird die Wechselwirkung durch von der Strahlungsaussendeeinrichtung 214 in den Innenvolumenbereich 120 des Gehäuses 110 ausgesendete Strahlung hervorgerufen und ein Teil dieser Strahlung ist nach der Wechselwirkung aus dem Innenvolumenbereich 120 zu der Signalempfangseinrichtung 210 gelangt.

Eine optische Wechselwirkung, die eine für die Identität oder den Zustand der Dialysatorvorrichtung 100 bzw. des Bluts im Blutbereich 130 bzw. des Dialysats im Dialysatbereich 170 charakteristische Information liefert, kann eine der folgenden sein:
- Reflexion von von der Strahlungsaussendeeinrichtung 240 ausgesendeter optischer Strahlung (d.h.Licht) an einer Grenzfläche zwischen der Wand 112 des Gehäuses 110, beispielsweise einem Fensterbereich 114, 116, 118 (siehe Fig. 2) und dem Dialysat oder dem Blut,
- Reflexion von von der Strahlungsaussendeeinrichtung 240 ausgesendeter optischer Strahlung an einer Grenzfläche zwischen der Membranfiltereinrichtung 190 und dem Dialysat oder dem Blut,
- Transmission von von der Strahlungsaussendeeinrichtung 240 ausgesendeter, optischer Strahlung mit einer Messwellenlänge, beispielsweise im infraroten Spektralbereich, die von einem in dem Dialysat und/oder in dem Blut enthaltenen Stoff absorbiert werden kann und die auf ihrem Weg zu der Strahlungsempfangseinrichtung 210 einen Strahlungslaufweg durch das Dialysat und/oder durch das Blut zurückgelegt hat,
- Emission von Lumineszenz- oder Fluoreszenzstrahlung durch einen in dem Dialysat und/oder in dem Blut enthaltenen Stoff, wobei eine entsprechende Lumineszenz- oder Fluoreszenzreaktion in diesem Stoff durch von der Strahlungsaussendeeinrichtung 240 ausgesendete optische Strahlung, z.B. UV Licht, hervorgerufen worden ist,
- Brechung von von der Strahlungsaussendeeinrichtung 240 ausgesendeter optischer Strahlung an einer Grenzfläche zwischen der Wand 112 des Gehäuses 110, z.B. einem Fensterbereich 114, 116, 118 (siehe Fig. 2) und dem Dialysat oder dem Blut, wobei die ausgesendete Strahlung z.B. unter einem zwischen 0° und 180° oder 0° und 90° liegenden Einfallswinkel auf die Grenzfläche auftrifft,
- Streuung von von der Strahlungsaussendeeinrichtung 240 ausgesendeter optischer Strahlung an einem in dem Dialysat oder in dem Blut enthaltenen Stoff, wobei dies auch z.B. eine so genannte dynamische Streuung, inkl. z.B. Raman-Streuung, von z.B. polychromatischem oder monochromatischem Licht wie etwa dichromatischer, polychromatischer oder monochromatischer Laserstrahlung einschließt; hierbei kann zur Auswertung einer dynamischen Streuung eine Analyse des Zeitverhaltens des gestreuten Lichts durchgeführt werden; die Messung ist hierbei nicht auf die Auswertung der Intensität beschränkt - auch die Phase der elektromagnetischen Strahlung und deren Zeitverhalten kann Informationen enthalten und zusätzlich oder anstelle der Intensitätserfassung erfasst und ausgewertet werden, dabei kann auch das zeitliche Abklingverhalten von Impulsen ausgewertet werden; - Wechselwirkung von von der Strahlungsaussendeeinrichtung 240 ausgesendeter optischer Strahlung mit einer passiven Identifizierungseinrichtung 192, die an der Dialysatorvorrichtung 100 oder an der Membranfiltereinrichtung 190 angebracht ist, wie etwa einem Barcode- oder einem Farbencode-Feld, wobei die Identifizierungseinrichtung 192 ein für die Identifizierung der Dialysatorvorrichtung 100 oder der Membranfiltereinrichtung 190 charakteristisches Identifizierungsmerkmal aufweist. Derartige Anwendungen wurden oben beschrieben.

Mit Verweis nun auf die Figuren 2 und 3 kann die Sensorvorrichtung 200 gemäß dem dargestellten Ausführungsbeispiel als ein Cliparm bzw. als eine Clipmanschette 210 ausgebildet sein, der bzw. die direkt an dem Gehäuse 110 der Dialysatorvorrichtung 100 lösbar und operativ verbindbar, beispielsweise aufclipbar, befestigt werden kann. In den in den Figuren 2 und 3 gezeigten Ausführungsformen umfasst die Sensorvorrichtung 200 zwei Cliparme bzw. Clipmanschetten 210, 210', die das beispielsweise zylinderförmige Gehäuse 110 umgreifen und zwischen sich aufnehmen können.

In einer Ausführungsform, wie in Fig. 2 gezeigt, kann ein Cliparm bzw. eine Clipmanschette 210 relativ zu einer in einer Längsrichtung 102 des Gehäuses 110 der Dialysatorvorrichtung 100 gemessenen Länge eine geringere Länge aufweisen, und entsprechend an dem Gehäuse 110 in der Längsrichtung 102 verschiebbar sein. In dieser Ausführungsform kann mit der Sensorvorrichtung 200 ein gewünschter Parameter in einer entlang der Längsrichtung 102 des Gehäuses 110 definierbaren Position oder auch, z.B. mit sequenziellen Messungen, an verschiedenen Positionen entlang der Längsrichtung 102 die Variation eines Parameters entlang der Längsrichtung 102 des Gehäuses 110 gemessen werden.

In einer alternativen Ausführungsform, wie in Fig. 3 gezeigt, kann ein Cliparm bzw. eine Clipmanschette 210 der Sensorvorrichtung 200 relativ zu der Längsrichtung 102 des Gehäuses 110 eine der Länge des Gehäuses 110 vergleichbare Abmessung aufweisen und entsprechend entlang der Längsrichtung 102 nur unwesentlich oder gar nicht verschiebbar ausgebildet sein.

Allgemein und auch insbesondere in den in den Figuren 2 und 3 gezeigten Ausführungsformen kann es vorteilhaft sein, wenn die Sensorvorrichtung 200 in einer oder mehreren, wiederholbaren bzw. wiedereinstellbaren, vorbestimmten relativen Positionen bzw.

Orientierungen in Bezug auf die Dialysatorvorrichtung 100 an dieser angebracht werden kann. Zu diesem Zweck umfasst das Gehäuse 110 der Dialysatorvorrichtung 100 an seiner Außenoberfläche mindestens einen ersten Koppelbereich 110, oder z.B. zwei sich an dem Gehäuse 110 im Wesentlichen gegenüberliegend angeordnete erste Koppelbereiche 111 und 111', der/die an vorbestimmten Positionen an der Vorrichtung 100 angeordnet sind. Entsprechend umfasst die Sensorvorrichtung 200 an einer dem Gehäuse 110 zugewandten Seite, z.B. in den Figuren 2 und 3 an einer Innenseite des Cliparms bzw. der Clipmanschette 210, einen zweiten Koppelbereich 211, oder z.B. zwei Koppelbereiche 211 und 211 die in einem mit der Vorrichtung 100 verbundenen Zustand der Sensorvorrichtung 200 im Wesentlichen kongruent zu dem bzw. den ersten Koppelbereich/en 111, 111' an dem Gehäuse 110 angeordnet sind. In der in Fig. 2 gezeigten Ausführungsform können an der Außenoberfläche des Gehäuses 110 mehrere in Längsrichtung 102 des Gehäuses 110, z.B. gleichmäßig beabstandete erste Koppelbereiche 111 vorgesehen sein. Diese ermöglichen, die Sensorvorrichtung 200 an verschiedenen vorbestimmten Positionen entlang der Längsrichtung 102 des Gehäuses 110 lösbar anzubringen. Die zweiten Koppelbereiche 211, 211' sind dazu ausgebildet, mit den ersten Koppelbereichen 111, 111' einzugreifen und zusammenzuwirken, so dass die Position der Sensorvorrichtung 200 in Bezug auf das Gehäuse 110 der Vorrichtung 100 wohl definiert und vorbestimmt ist.

In einer Ausführungsform umfassen die ersten Koppelbereiche 111, 111' und die zweiten Koppelbereich 211,211' jeweils einander anziehende Magnete. In einer anderen Ausführungsform umfassen die ersten Koppelbereiche 111, 111' sogenannte weibliche Aufnahmeelemente und die zweiten Koppelbereiche 211, 211' sogenannte männliche Steckelemente, die zur lösbaren Aufnahme in den weiblichen Aufnahmeelementen ausgebildet sind. Vorzugsweise sind dabei die weiblichen Aufnahmeelemente den ersten Koppelbereichen 111, 111' des Gehäuses 110 der Dialysatorvorrichtung 100 und die männlichen Steckelemente den zweiten Koppelbereichen 211, 211 der Cliparme bzw. Clipmanschetten 210, 210' der Sensorvorrichtung 200 zugeordnet.

Bei einem, mehreren oder allen Ausführungsbeispielen kann die Sensorvorrichtung 200 und/oder die Dialysatorvorrichtung 100 eine spezielle Form aufweisen, z.B. eine oder mehrere Einbuchtungen, Vertiefungen oder Vorsprünge am oder im Dialysatorgehäuse, die nur dort eine zentrierte Anbringung des Clips, d.h. der Sensorvorrichtung erlauben.

Alternativ oder zusätzlich zu der hierüber beispielhaft beschriebenen Ausgestaltung der lösbaren, operativen Verbindbarkeit der Sensorvorrichtung 200 an der Dialysatorvorrichtung 100 kann diese auch durch die folgenden, mechanisch lösbaren, insbesondere formschlüssig befestigbaren Mechanismen bewirkt werden:
- Die Sensorvorrichtung 200 ist als eine Clipeinrichtung mit mindestens einem oder auch zwei (wie in den Figuren 2 und 3 gezeigt) oder mehreren flexiblen Cliparmen bzw. Clipmanschetten 210, 210' ausgebildet, die dazu ausgebildet sind, das Gehäuse 110 der Dialysatorvorrichtung 100 in einem Winkelbereich von mehr als 180°, vorzugsweise mehr als 270° und noch mehr bevorzugt mehr als 320° zu umgreifen.
- Die Sensorvorrichtung 200 umfasst einen Grundkörper (nicht gezeigt) und eine Clipeinrichtung mit mindestens einem Paar von an dem Grundkörper befestigten, elastisch-flexiblen Cliparmen bzw. Clipmanschetten oder an dem Grundkörper elastisch-flexibel angelenkten Cliparmen bzw. Clipmanschetten (nicht gezeigt), die dazu ausgebildet sind, zwischen sich das Gehäuse 110 bzw. einen Bereich des Gehäuses 110 aufzunehmen. Aufgrund ihrer elastisch-flexiblen Ausgestaltung oder Anlenkung können die Cliparme bzw. Clipmanschetten das Gehäuse 110 oder einen Teil des Gehäuses 110 clipartig umklammern.
- Die Sensorvorrichtung 200 umfasst ein erstes Glied (nicht gezeigt) einer Verbindungseinrichtung (nicht gezeigt), wie etwa eine Schwalbenschwanzeinrichtung, eine Schraube bzw. ein Klettband, und die Dialysatorvorrichtung 100 umfasst ein zweites Glied bzw. Gegenglied (nicht gezeigt) dieser Verbindungseinrichtung, wie etwa eine Schwalbenschwanzaufnahmeeinrichtung, ein zum Einschrauben der Schraube ausgebildetes Loch oder Schraubengewinde bzw. ein Gegenstück zu dem Klettband. Dabei ist das erste Glied in das zweite Glied oder das zweite Glied in das erste Glied lösbar formschlüssig eingreifbar ausgebildet.
- Die Sensorvorrichtung 200 wird mittels einer elastischen Spanneinrichtung (nicht gezeigt), wie etwa einem Gummiring, an dem Gehäuse 110 festgespannt. Dabei kann die Spanneinrichtung dazu ausgebildet sein, in einem elastisch gespannten Zustand die Sensorvorrichtung 200 und das Gehäuse 110 der Dialysatorvorrichtung zu umspannen.
- Die Sensorvorrichtung 200 umfasst mindestens ein Paar von Armen bzw. Manschetten, die jeweils einen distalen Endbereich mit einem dort vorgesehenen ersten Glied einer Haken- oder Eingreifeinrichtung aufweisen. Dabei sind die Arme bzw. Manschetten dazu ausgebildet, das Gehäuse 110 in einem Winkelbereich von mehr als 180°, vorzugsweise mehr als 270° und mehr bevorzugt mehr als 320° zu umgreifen. Ferner ist eine elastisch spannbare Spanneinrichtung mit zwei gegenüberliegenden Endabschnitten vorgesehen, an denen jeweils eine zum lösbaren Eingreifen mit einem ersten Glied der Haken- oder Eingreifeinrichtung ausgebildetes, zweites Glied bzw. Gegenglied der Haken- oder Eingreifeinrichtung vorgesehen ist. Dabei kann in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung 200 eingreifen, so dass die Arme bzw. Manschetten der Sensorvorrichtung 200 zusammen mit der elastisch gespannten Spanneinrichtung das Gehäuse 110 vollständig umringen bzw. umspannen. Optional kann die Sensorvorrichtung 200 einen Grundkörper (nicht gezeigt) umfassen, an dem die Arme bzw. Manschetten angelenkt sind oder mit dem Arme bzw. Manschetten elastisch-flexibel verbunden sind.
- Die Sensorvorrichtung 200 ist dazu ausgebildet, z.B. in einer Vertiefung, in bzw. an der Wand 112 des Gehäuses 110 der Dialysatorvorrichtung 100 lösbar integriert zu werden. Diese Ausführungsform ist insbesondere dann geeignet, wenn die Sensorvorrichtung 200 eine räumlich kleine Ausdehnung aufweist, wie etwa im Fall einer RF-Identifizierungsausleseeinrichtung 212.

In der in Fig. 2 gezeigten Ausführungsform einer erfindungsgemäßen Vorrichtung mit einer Sensorvorrichtung 200 ist das Gehäuse 110 der Dialysatorvorrichtung für optische Strahlung (d.h. Licht) nicht ausreichend durchlässig bzw. transparent. Daher sind in der Wand 112 des Gehäuses 110 sogenannte Fensterbereiche 114, 116, 118 vorgesehen, die für optische Strahlung ausreichend durchlässig bzw. transparent sind. In der Clipmanschette 210 ist eine Strahlungsaussendeeinrichtung 240 integriert, die eine erste, z.B. optische oder sonstige, Strahlungsquelle 242 zum Aussenden von elektromagnetischer Strahlung, z.B. Licht, in den Blutbereich 130 und eine zweite, z.B. optische, Strahlungsquelle 244 zum Aussenden von elektromagnetischer Strahlung, z.B. Licht in den Dialysatbereich 170 umfasst. Integriert in den Cliparm bzw. die Clipmanschette 210, 210' ist ferner eine Strahlungsmesseinrichtung 220 mit einem ersten, z.B. optischen Strahlungsdetektor 222, der zum Detektieren von aus dem Blutbereich 130 austretender elektromagnetischer Strahlung, z.B. Licht, ausgebildet ist, und einem zweiten, z.B. optischen, Strahlungsdetektor 224, der zum Detektieren von aus dem Dialysatbereich 170 austretender elektromagnetischer Strahlung, z.B. Licht, ausgebildet und entsprechend angeordnet ist. Dabei ist der erste Strahlungsdetektor 222 dazu ausgebildet, aus dem Blutbereich 130 austretender elektromagnetischer Strahlung, z.B. Licht zu messen, das von der ersten Strahlungsquelle 242 dort hinein gestrahlt worden ist. Der zweite Strahlungsdetektor 224 ist dazu ausgebildet, aus dem Dialysatbereich 170 austretende elektromagnetische Strahlung, z.B. Licht zu detektieren, die von der zweiten Strahlungsquelle 244 dort hinein gestrahlt worden ist.

In einer Ausführungsform ist der erste Strahlungsdetektor 222 in Bezug auf die erste Strahlungsquelle 242 und den Blutbereich 130 so räumlich angeordnet bzw. positioniert, dass das von der ersten Strahlungsquelle 242 in den Blutbereich 130 ausgestrahlte Licht von dem zweiten Strahlungsdetektor 222 in einer Reflexionsanordnung, in der nur im Wesentlichen zurückgeworfenes bzw. rückwärts gestreutes Licht gemessen wird. In einer anderen Ausführungsform ist der erste Strahlungsdetektor 222 in Bezug auf die erste Strahlungsquelle 242 und den Blutbereich 130 in einer Transmissionsanordnung angeordnet, in der im Wesentlichen nur Licht gemessen wird, das Streuung in einer im Wesentlichen Vorwärtsrichtung oder Transmission in Richtung eines wohl definierten Laufwegs durch den Blutbereich 130 untergangen hat. In noch einer anderen Ausführungsform ist eine Streuanordnung realisiert, bei der ein Blickwinkel des ersten Strahlungsdetektors 222 in Bezug auf eine Hauptabstrahlrichtung der ersten Strahlungsquelle 242 unter einem Winkel, vorzugsweise einem Winkel von etwa 90°, jedenfalls einem Winkel der wesentlich von 0° und 180° verschieden ist, gemessen wird. Jeweils entsprechendes gilt für den zweiten optischen Strahlungsdetektor 224 und dessen Anordnung bzw. Position in Bezug auf die zweite Strahlungsquelle 244 und den Dialysatbereich 170.

In der in Fig. 3 gezeigten Ausführungsform einer Vorrichtung oder eines Systems gemäß der Erfindung ist die Sensorvorrichtung 200 so ausgebildet, dass sie das im Beispiel zylinderförmige Gehäuse 110 der Dialysatorvorrichtung 100 nahezu vollständig umgreift und überdeckt. Dies ist im Beispiel der Fig. 3 durch eine Ausbildung der Sensorvorrichtung 200 in Form einer Clipmanschette 210, 210' realisiert.

Im rechten Bereich der Fig. 3 ist die geöffnete, von dem Gehäuse 110 abgenommene und planar entrollte Innenseite der Sensorvorrichtung 200 gezeigt, die in einem an der Dialysatorvorrichtung 100 angebrachten Zustand dieser Vorrichtung 100 zugewandt ist. In der im Wesentlichen rechteckförmigen innenseitigen Fläche der Sensorvorrichtung 200 ist eine Vielzahl von Strahlungseintrittsbereichen 230, 232-x, 232-y, 236-x,y und eine Vielzahl von Strahlungsaustrittsbereichen 250, 252-x, 254-y, 256-x,y ausgebildet. Zur Bezeichnung und Definition von Richtungen ist für die entrollte Sensorvorrichtung 200 in Fig. 3 die Richtung der Längsachse 102 und die Richtung des Umfangs 104 des Gehäuses 110 der Dialysatorvorrichtung 100 mit gestrichelten Linien eingezeichnet.

In einer Ausführungsform bildet die Vielzahl der Strahlungseintrittsbereiche 230 eine z. B. in der Richtung des Umfangs 104 ausgerichtete, eindimensionale Anordnung 231 von Strahlungseintrittsbereichen 232-x, die sich über einen Teil des Umfangs oder bei einem oder mehreren Ausführungsbeispielen auch im Wesentlichen über die gesamte Ausdehnung entlang der Umfangsrichtung 104 der Sensorvorrichtung 200 erstrecken kann. In einer dazu alternativen oder zusätzlich realisierbaren Ausführungsform bildet eine Vielzahl der Strahlungseintrittsbereiche 230 eine zweite eindimensionale Anordnung 233 mit einer Vielzahl von Strahlungseintrittsbereichen 234-y, die sich über einen Teil oder bei einem oder mehreren Ausführungsbeispielen im Wesentlichen über die gesamte Ausdehnung der Sensorvorrichtung 200 entlang der Richtung der Längsachse 102 erstrecken kann. In einer weiteren Ausführungsform bilden die Strahlungseintrittsbereiche 230 eine zweidimensionale Anordnung 235 mit einer Vielzahl von Strahlungseintrittsbereichen 236-x,y, die in einer schräg oder parallel zu der Richtung des Umfangs 104 und der Richtung der Längsachse 102 ausgerichteten regelmäßigen Anordnung, etwa einer rechtwinkligen Gitteranordnung, oder auch in unregelmäßiger Form angeordnet sein können. Dabei erstreckt sich diese zweidimensionale Anordnung 235 bzw. Gitteranordnung über einen Teil der, oder bei einem oder mehreren Ausführungsbeispielen auch im Wesentlichen über die gesamte, Oberfläche der Sensorvorrichtung 200. Die Anordnung der Strahlungseintrittsbereiche muss sich nicht über die gesamte Sensorvorrichtung erstrecken, sondern kann auch nur in Teilbereichen vorhanden sein, die von besonderem Interesse sind.

In jedem Strahlungseintrittsbereich 230, 232-x, 232-y, 236-x,y ist entweder ein Strahlungsempfänger, insbesondere ein Lichtdetektor angeordnet, oder ein Eintrittsbereich eines optischen Lichtleiters bzw. einer Lichtleitfaser. Im letzten Fall leitet der Lichtleiter bzw. die Lichtleitfaser das in dem Strahlungseintrittsbereich in den Lichtleiter bzw. die Lichtleitfaser eingetretene Licht durch den Lichtleiter bzw. die Lichtleitfaser bis zu einem Strahlungsaustrittsbereich am entgegengesetzten Ende, wo ein Lichtdetektor vorgesehen ist, der das aus dem entgegengesetzten Ende des Lichtleiters bzw. der Lichtleitfaser austretende Licht detektiert. Jeder Lichtdetektor kann, je nach Messaufgabe und zu erwartender Lichtintensität bzw. Wellenlänge einer der Folgenden sein: eine Fotodiode, ein Fototransistor, ein CMOS-Lichtdetektor, ein Fotomultiplier oder eine Avalanche-Fotodiode. In der Ausführungsform mit Lichtleitern bzw. Lichtleitfasern können die Lichtaustrittsenden zu einer zu der Anordnung der Lichteintrittsöffnungen kongruenten Gitteranordnung zusammengefasst sein und das aus der Vielzahl dieser Austrittsöffnungen auftretende Licht auf einem entsprechend angeordneten bzw. ausgerichteten ein- oder zweidimensionalen Lichtdetektor, beispielsweise einem ein- oder zweidimensionalen CCD-Sensor, in seiner räumlichen Verteilung und bezüglich seiner Intensität gemessen werden.

Entsprechend der räumlichen Anordnung der Strahlungseintrittsbereiche 230 in eindimensionalen Anordnungen 231, 233 oder eine zweidimensionale Anordnung 235 kann auch die Vielzahl der Strahlungsaustrittsbereiche 250 in einer der folgenden Anordnungen ausgebildet sein: eine eindimensionale Anordnung 251 von Strahlungsaustrittsbereichen 252-x, die entlang der Richtung des Umfangs 104 verteilt angeordnet sind und sich im Wesentlichen über die gesamte Ausdehnung der entrollten Sensorvorrichtung 200 erstrecken, eine eindimensionale Anordnung 253 von Strahlungsaustrittsbereichen 254-y, die entlang der Richtung der Längsachse 102 verteilt angeordnet sind und sich im Wesentlichen über die gesamte Ausdehnung in dieser Richtung auf der entrollten Sensorvorrichtung 200 erstrecken, und/oder eine zweidimensionale Anordnung 255 von einer Vielzahl von Strahlungsaustrittsbereichen 256-x,y, die in einer entlang der Richtung des Umfangs 104 und der Längsachse 102 orientierten, im Wesentlichen rechtwinkligen Gitteranordnung angeordnet sind. In jedem Strahlungsaustrittsbereich 250 kann entweder eine Lichtquelle, wie etwa eine LED, oder eine Austrittsöffnung für einen Austritt von Licht aus einem/r Licht zuführenden Lichtleiter bzw. Lichtleitfaser angeordnet sein. In der Ausführungsform mit Licht zuführenden Lichtleitern bzw. Lichtleitfasern kann am gegenüberliegenden Ende eines/r jeweiligen Lichtleiters bzw. Lichtleitfaser eine gesonderte Lichtquelle angeordnet sein. Alternativ dazu können die Strahlungsaustrittsbereiche 250 auch in einer beispielsweise zu der Gitterstruktur der Strahlungseintrittsbereiche 230 kongruenten Gitteranordnung zusammengefasst sein und von mehreren Strahlungsquellen oder auch einer einzigen Lichtquelle, die z.B. ein homogenes Strahlungsfeld bzw. paralleles Licht abstrahlt, angestrahlt werden, wie etwa ein aufgeweiteter Laserstrahl. Auch andere Lichtquellen können verwendet werden, wie z.B. eine oder mehrere Halogenlampen oder sonstige Lampen, Fluoreszenzlichtquellen oder dgl. Wenn mehrere Lichtquellen verwendet werden, sind die Lichtquellen bei einem, mehreren oder allen Ausführungsbeispielen einzeln schaltbar, so dass eine gezielte, gesteuerte Bestrahlung durchführbar ist.

Durch selektive Ansteuerung bzw. Strahlungsemission aus den Strahlungsaustrittsbereichen 250 können, wie ein Fachmann leicht erkennt, unterschiedliche Lichteinstrahlungsverteilungen bzw. Beleuchtungsgeometrien realisiert werden. Auch können durch selektive Messung von Strahlungsintensitäten in selektiv ausgewählten Strahlungseintrittsbereichen 230 verschiedenartige Abstrahl- bzw. Ausstrahlkonfigurationen von Licht aus dem Gehäuse 110 der Dialysatorvorrichtung 100 realisiert werden.

In der Ausführungsform, bei der Strahlung von den Strahlungseintrittsbereichen durch einen Lichtleiter bzw. eine Lichtleitfaser zu einem räumlich entfernten Detektor geleitet wird, kann hinter dem Lichtaustrittsbereich aus Lichtleiter bzw. Lichtleitfaser auch eine Lichtmesseinrichtung vorgesehen sein, die eine Wellenlängenauswahleinrichtung, wie etwa einen Monochromator, und einen dahinter angeordneten Lichtdetektor umfassen kann. Die Wellenlängenauswahleinrichtung kann dazu ausgebildet sein, einen schmalen Wellenlängenbereich, der eine vorbestimmte Nachweis- bzw. Messwellenlänge umfasst, auszuwählen.

Beispielsweise kann ein schmalbandiger Wellenlängenbereich von Messwellenlängen so realisiert bzw. abgetastet werden, dass ein Lumineszenz- oder Fluoreszenzemissionsspektrum erfasst werden kann. Ein Fachmann weiß, dass ein Lumineszenz-, Fluoreszenz- oder Fluoreszenzemissionsspektrum charakteristisch ist für einen ausgewählten Stoff, der beispielsweise im Blutbereich 130 bzw. im Blut gelöst ist, oder der im Dialysatbereich 170 bzw. im Dialysat gelöst ist. Hierbei kann bei einem oder mehreren Ausführungsbeispielen auf dem Gehäuse ein solches lumineszierendes oder fluoreszierendes Material angebracht sein, das zur Identifikation des Dialysators dienen kann. Damit kann der Typ des gerade eingesetzten, verwendeten oder zu verwendenden Dialysators erkannt werden.

In Fig. 4 ist veranschaulicht, wie eine Dialysatorvorrichtung 100 gemäß dem dritten Aspekt der Erfindung und eine Sensorvorrichtung 200 gemäß dem zweiten Aspekt der Erfindung in Bezug auf die Steuerung bzw. Auslesung und Führung von Mess- und Steuersignalen in einer Dialysemaschine 300 gemäß dem vierten Aspekt der Erfindung integriert bzw. an dieser angeschlossen sein kann. Die in Fig. 4 gezeigte Dialysemaschine 300 umfasst die in Fig. 1 gezeigten Komponenten des Dialysatkreislaufs 40 und des Blutkreislaufs, die in Fig. 4 nicht im Einzelnen dargestellt sind.

Die Dialysatorvorrichtung 100 ist über den in Fig. 1 gezeigten Dialysatzufluss 72 und den Dialysatabfluss 78 fluidtechnisch in den Dialysatkreislauf 40 der Dialysemaschine 300 eingebunden. Ferner ist die Dialysatorvorrichtung 100 über das arterielle Schlauchsystem 24 und das venöse Schlauchsystem 34 mit dem arteriellen Zugang 22 und dem venösen Zugang 36 des Patienten 10 verbunden, siehe Fig. 1.

Die in Fig. 4 gezeigte Dialysemaschine 300 umfasst ein Maschinengehäuse 302, in dem die in Fig. 1 gezeigten Komponenten des Dialysatkreislaufs 40 angeordnet sind, ausgenommen die Dialysatorvorrichtung 100. Diese kann innerhalb des Maschinengehäuses 302 oder extern dazu angeordnet sein. Bei externer Anordnung ist die Dialysatorvorrichtung 100 über die Dialysatzufuhr 72 und den Dialysatablass 78 in Fluidkommunikation mit den innerhalb des Maschinengehäuses 302 angeordneten Komponenten des Dialysatkreislaufs 40, siehe Fig. 1. Das Maschinengehäuse 302 umfasst Gehäuserollen 304, 304', mittels der die Maschine 300 auf einem Boden rollbar ist. Auf dem Maschinengehäuse 302 angeordnet oder beabstandet dazu und mit den entsprechenden Datenleitungen angeschlossen bereitgestellt ist eine Datenausgabe- bzw. Visualisierungseinrichtung 310 und eine Dateneingabeeinrichtung (nicht gezeigt). Die Dateneingabeeinrichtung und Datenausgabe- bzw. Visualisierungseinrichtung 310 bilden zusammen eine Eingabe-/Ausgabeeinrichtung, die in jeglicher hinsichtlich ihrer Vielfältigkeit dem Fachmann bekannten Formen moderner Mensch-Maschine-Schnittstellen ausgestaltet sein kann.

Die Dialysemaschine 300 umfasst auch eine Steuereinheit 320, die zum Steuern der Komponenten des Dialysatkreislaufs 40, der Strahlungsaussendeeinrichtung 210 und deren Komponenten sowie zur Steuerung der Strahlungsmesseinrichtung 220 und deren Teilkomponenten ausgebildet ist, eine Datenverarbeitungs- bzw. Auswerteeinheit 330, die dazu ausgebildet ist, die von der Sensorvorrichtung 200 erzeugten Messsignale aufzunehmen, umzurechnen in relevante Parameter, auszuwerten und zur Speicherung bzw. Datensicherung bereitzustellen, und eine Speichereinheit 340, die dazu ausgebildet ist, von der Datenverarbeitungs- bzw. Auswerteeinheit 330 bereitgestellte Messdaten bzw. ausgewertete Informationen aufzunehmen und zu speichern und insbesondere ferner Computer-Programme, die die Steuereinheit 320, die Datenverarbeitungs- bzw. Auswerteeinheit 330 und/oder die Eingabe-/Ausgabeeinrichtung 210 steuern, zu speichern.

Die Dialysemaschine 300 umfasst ferner ein Datenbussystem 360, an dem die steuerbaren Komponenten des Dialysatkreislaufs 40 und des Blutkreislaufs 20, die in Fig. 4 gezeigten Einheiten 310, 320, 330, 340 sowie die Dialysatorvorrichtung 100 und die daran lösbar anbringbare Sensorvorrichtung 200 angeschlossen und kommunikationstechnisch miteinander verbunden sind. Das Datenbussystem 360 umfasst eine Eingabe-/Ausgabedatenleitung 362, mittels der die Eingabe-/Ausgabeeinrichtung 310 kommunikationstechnisch angeschlossen ist, eine Steuerleitung 364, mittels der die Steuereinheit 320 kommunikationstechnisch angeschlossen und in die Lage versetzt ist, Daten von der Eingabe-/Ausgabeeinrichtung 310 zu empfangen und Steuerdaten bzw. ausgewertete Daten zu der Eingabe-/Ausgabeeinrichtung 310, etwa zur Ausgabe bzw. Anzeige, zu übertragen, eine Betriebsdatenleitung 366, mittels der die Steuereinheit 360 und die Datenverarbeitungs- bzw. Auswerteeinheit 330 kommunikationstechnisch miteinander verbinden sind, eine Datenspeicherleitung 368, mittels der die Speichereinheit 340 und die Datenverarbeitungs- bzw. Auswerteeinheit 330 kommunikationstechnisch miteinander verbunden sind, eine Dialysatorvorrichtungssignalleitung 370, mittels der die Dialysatorvorrichtung 100 und die Dialysemaschine 300 kommunikationstechnisch miteinander verbunden sind, und eine Sensorvorrichtungssignalleitung 372, mittels der die Sensorvorrichtung 200 und die Datenverarbeitungs- bzw. Auswerteeinheit 330 Dialysemaschine 300 kommunikationstechnisch miteinander verbunden sind.

Die Dialysatorvorrichtung 100 ist mittels der Dialysatorvorrichtungssignalleitung 370 mit der Steuereinheit 320 und die an der Dialysatorvorrichtung 100 lösbar anbringbare Sensorvorrichtung 200 mittels der Sensorvorrichtungssignalleitung 372 mit der Datenverarbeitungs- bzw. Auswerteeinheit 330 der Dialysemaschine 300 kommunikationstechnisch verbunden. Über die Sensorvorrichtungssignalleitung 372 kann die Datenverarbeitungs- bzw. Auswerteeinheit 330 von der Sensorvorrichtung 200 erzeugte Messdaten, z.B. gemessene Strahlungsintensitätsdaten oder Identifizierungsdaten, zu der Datenverarbeitungs- bzw. Auswerteeinheit 330 zur Verarbeitung und Auswertung übertragen und auch von der Einheit 330 Steuerungsdaten zum Ansteuern der Strahlungsaussendeeinrichtung/en 240 empfangen. Über die Dialysatorvorrichtungssignalleitung 370 kann die Dialysatorvorrichtung 100 von der Steuereinheit 320 Steuersignale empfangen und umgekehrt für ihren Zustand bzw. ihre Identifizierung charakteristische Information an die Steuereinheit 320 übertragen.

Die in Fig. 4 gezeigte Dialysemaschine 300 umfasst ferner einen ersten Gehäusehalter 180 und einen zweiten Gehäusehalter 182. Diese sind dazu ausgebildet, zwischen sich die Dialysatorvorrichtung 100 lösbar einsetzbar zu tragen. Der erste Gehäusehalter 180 und ggf. der zweite Gehäusehalter 182 können als Endkappe zum lösbaren Aufsetzen auf ein jeweiliges Ende der Dialysatorvorrichtung 100 ausgebildet und dabei insbesondere wiederverwendbar sein, während die Dialysatorvorrichtung 100 als Ganzes als Einmalartikel ausgebildet ist. Dabei dienen die Gehäusehalter 180, 182 zur fluidtechnischen Einbindung der Dialysatorvorrichtung 100 in den Dialysatkreislauf 40 und den Blutkreislauf 20. Die Sensorvorrichtung 200 kann mit dem ersten oder zweiten Gehäusehalter 180, 182 dauerhaft verbunden sein oder auch getrennt hiervon ausgebildet sein.

In einer Ausführungsform ist der erste Gehäusehalter 180 fest mit dem Gehäuse 110 der Dialysemaschine 300 verbunden, und der zweite Gehäusehalter 182 ist als gesondertes Teil ausgebildet und an der Dialysatorvorrichtung 100 lösbar, z.B. anclipbar, und ebenso an dem Maschinengehäuse 302 lösbar, z.B. anclipbar ausgebildet. In einer anderen Ausführungsform ist nur ein einziger Gehäusehalter 180 vorgesehen, der die Dialysatorvorrichtung 100 lösbar, z.B. anclipbar, und zuverlässig trägt. In einer noch anderen Ausführungsform werden die Dialysatorvorrichtung 100 und die Gehäusehalter 180 und 182 abgesetzt von der Dialysemaschine 300, beispielsweise in der Nähe des zu behandelnden Patienten 10, eingesetzt.

Es ist für einen Fachmann offensichtlich, dass die Sensorvorrichtung 200, insbesondere in den bezüglich der Figuren 2 und 3 beschriebenen Ausführungsformen als Ganzes so ausgebildet sein kann, dass die folgenden Funktionen bzw. konkretisierten Ausgestaltungen optischer Messungen an der Dialysatorvorrichtung 100 durchgeführt werden können. Konkret kann die Funktionalität der in Fig. 3 gezeigten Sensorvorrichtung 200 z.B. für folgende Aufgaben angepasst werden:
(i) Messungen im Blutbereich 130 zur Bestimmung der Konzentrationen von Stoffen wie etwa von Albumin, anderen Stoffen oder urämischen Toxinen im Blut,
(ii) Messung von physikalischen Parametern des Bluts im Blutbereich 130 bzw. des Dialysats im Dialysatbereich 170, z.B. die Viskosität und/oder die Strömungsgeschwindigkeit des Dialysats bzw. des Bluts oder die Hämatokritkonzentration im Blut, und
(iii) Messungen im Dialysatbereich 170 zur Bestimmung der Konzentration von Stoffen wie etwa von Proteinen, anderen Stoffen oder urämischen Toxinen im Dialysat.

Im Folgenden werden Ausführungsbeispiele für mit der Sensoreinrichtung 200 ausführbare optische Messungen beschrieben.

Bei einem, mehreren oder allen Ausführungsbeispielen können Messungen an der Dialysatorvorrichtung durchgeführt werden. Dies wird im Folgenden näher dargelegt.

In die mit Verweis auf die Fig. 2 beschriebene Sensoreinrichtung 200 eingebaut oder z.B. über Lichtleitfasern bzw. Lichtleiter optisch damit verbunden ist eine Lichtquelle vorgesehen, die schmalbandiges Licht aussendet, z.B. eine LED, ein Laserstrahl oder ein/e polychromatisches oder monochromatisches Licht führende/r Lichtleiter bzw. Lichtleitfaser. Diese Lichtquelle strahlt, eventuell über ein strahlformendes Element, z.B. eine Sammellinse, senkrecht oder in einem vorbestimmten Winkel durch eine transparente Stelle, z.B. durch einen Fensterbereich 114, 116, 118 Licht in den Innenvolumenbereich 120 des Gehäuses 110 ein. Das eingestrahlte Licht trifft in den Bereich der Dialysatorvorrichtung 100, in dem die Membran der Membranfiltereinrichtung 190 mit ihren Membranfasern angeordnet ist. Räumlich benachbart zu der Lichtquelle in der Sensorvorrichtung 200 integriert oder über eine/n Lichtleiter bzw. Lichtleitfaser optisch damit verbunden ist ein Lichtdetektor, der in seinem Blickfeld gestreutes oder reflektiertes Licht einfängt, das z.B. an dem Gehäuse, an der Grenzfläche zwischen dem Gehäuse und der Flüssigkeit oder in dem von der Lichtquelle bestrahlten Bereich des Innenvolumenbereichs 120 reflektiert wird. Der Lichtdetektor kann dabei eine einzelne oder mehrere Photodioden oder auch ein anderer Detektortyp, wie etwa ein Phototransistor, ein CMOS-Detektor, ein CCD, ein Photomultiplier oder eine Avalanche-Photodiode sein.

Verteilt über der Innenseite der Sensorvorrichtung 200 angeordnet ist eine eindimensionale Anordnung 231, 233 oder eine zweidimensionale Anordnung 235 von Lichtdetektoren bzw. Lichteintrittsöffnungen (Strahlungseintrittsbereichen 230) mit den Eintrittsenden von Lichtleitern bzw. Lichtleitfasern, deren Lichteintrittfacetten in die Blickrichtung zeigen, aus der die zu analysierende Lichtstrahlung kommt. Die Lichtleiter bzw. Lichtleitfasern dienen dazu, das zu analysierende Licht aus der Sensorvorrichtung 200 herauszuleiten und an anderer Stelle z.B. mittels der vorgenannten Lichtdetektoren, gegebenenfalls in Kombination mit Wellenlängenauswahleinrichtungen (z.B. Monochromator/en), zu analysieren. Durch Auswahl der Messwellenlänge lässt sich einstellen, mit welchen im Dialysat und/oder im Blut gelösten Stoffen, das analysierte Licht wechselgewirkt hat. Der Eintritt und/oder Austritt des Lichts kann auch unter einem Winkel zur Gehäuseoberfläche erfolgen, wobei die Lichtdetektoren oder Lichtleiter hierbei schräg zur Gehäuseoberfläche angeordnet sein können.

Beispielsweise findet im grünen Spektralbereich (500 - 600 nm) die Wechselwirkung von Licht vor allem mit dem Hämoglobin im Blut statt. Hämoglobin weist in diesem Spektralbereich Absorptionsbanden auf. Im infraroten Spektralbereich absorbiert z.B. im Blut enthaltene Glucose (etwa bei der Wellenlänge 2,3 µm) und Harnstoff (etwa bei einer Wellenzahl (reziproke Wellenlänge) im Bereich von ca. 4500 bis 4700 cm⁻¹).

In den Strahlungseintrittsbereichen 230 der Sensorvorrichtung 200 können auch eine oder mehrere Strahlungsmesseinrichtungen 220 mit einer eine Fokus- bzw. Fokalrichtung aufweisenden Messoptikeinrichtung und einem eine lichtsensitive Detektorfläche aufweisenden Lichtdetektor angeordnet sein. Dabei kann die Messoptikeinrichtung dazu ausgebildet sein, einen Raumbereich aus dem Innenvolumenbereich 120, z.B. aus dem Blutbereich 130 oder dem Dialysatbereich 170, auf die Detektorfläche des Lichtdetektors abzubilden, wobei der abgebildete Raumbereich durch Einstellen der Fokaltiefe und der Fokalrichtung definiert ist. Durch Änderung der Fokaltiefe und gegebenenfalls zusätzlich durch Änderung der Fokaltiefe des detektierten Lichts kann der Bereich, in dem eine optische Wechselwirkung stattfindet, im Innenvolumenbereich 120 ausgewählt werden. Durch Auswahl der Messwellenlänge kann ein spezifischer nachzuweisender Analyt ausgewählt werden.

Bei den Wechselwirkungen Reflexion, Absorption und Transmission gleicht die Wellenlänge des analysierten Lichts der Wellenlänge der eingestrahlten Lichtstrahlung. Die Lichtstrahlung kann bei einem oder mehreren Ausführungsbeispielen im Wesentlichen senkrecht auf die Gehäusewand 112 der Dialysatorvorrichtung 100 eingestrahlt und in der gleichen Richtung, oder auch rechtwinklig (oder nahezu rechtwinklig) dazu analysiert werden, z.B. bei Ausnutzung von Fluoreszenzeigenschaften. In dieser Messanordnung lässt sich aus einer geringen analysierten Lichtintensität auf das Vorliegen einer starken Absorption schließen, was wiederum Rückschlüsse auf die Konzentration eines z.B. Absorption verursachenden Stoffes oder einer Stoffmischung ermöglicht. So kann eine zeitliche Veränderung und eine räumliche Verteilung der Absorption erfasst und damit eine räumliche Verteilung und/oder eine zeitliche Veränderung einer Stoffkonzentration gemessen werden.

Bei einem, mehreren oder allen Ausführungsbeispielen können alternativ oder zusätzlich Fluoreszenz- bzw. Lumineszenzmessungen an der Dialysatorvorrichtung durchgeführt werden. Dies wird im Folgenden näher dargelegt.

Ist die Wellenlänge der analysierten Lichtstrahlung eine andere, z.B. eine längere, als die Wellenlänge der eingestrahlten Lichtstrahlung, so können mit der zuvor beschriebenen Messanordnung, mit einer Streuanordnung der Blickrichtung des Lichtdetektors in Bezug auf die Einstrahlrichtung der Lichtquelle, bei entsprechender Intensität und Auswahl der Wellenlänge der eingestrahlten Lichtstrahlung, z.B. im ultravioletten (UV) Spektralbereich, Fluoreszenz- bzw. Lumineszenzwechselwirkungen mit im Blut oder im Dialysat gelösten Stoffen induziert, gemessen und gegebenenfalls auch quantifiziert werden. Dabei ist es bei einem oder mehreren Ausführungsbeispielen möglich, die die Wechselwirkung anregende Lichtstrahlung in zeitlich kurzen, z.B. hoch-intensiven Lichtpulsen einzustrahlen und das infolge der Fluoreszenz- bzw. Lumineszenzreaktion erzeugte Licht zeitlich und/oder spektral aufgelöst zu detektieren. Derartige Fluoreszenz- bzw. Lumineszenzmessungen ermöglichen Rückschlüsse auf im Dialysat bzw. im Blut gelöste Stoffe sowie deren Konzentration und deren räumliche Verteilung. Dies ermöglicht eine zeitaufgelöste Messung, bei der die Abklingzeit der Fluoreszenz als Charakteristikum gemessen wird.

Anstelle von Pulsen kann auch eine kontinuierliche Bestrahlung vorgesehen sein.

Der Detektor ist bei einem oder mehreren Ausführungsbeispielen in einem Winkel, z.B. in einem rechten Winkel, zur Lichteinfallsrichtung angeordnet, so dass die Messungen durch Reflektionen kaum oder gar nicht gestört werden.

Bei einem, mehreren oder allen Ausführungsbeispielen kann eine Brechung von Lichtstrahlung an der Dialysatorvorrichtung ausgewertet werden. Dies wird im Folgenden näher erläutert.

Die möglichen Ausgestaltungen der Lichteinstrahlung und der Lichtdetektion aus dem Beispiel 1 gelten hier entsprechend. Konkret lässt sich damit eine Messung von Lichtbrechung an der Dialysatorvorrichtung 100 wie folgt realisieren:
Eingestrahlte Lichtstrahlung wird mit einer wohl definierten Richtung nicht senkrecht auf die Gehäusewand 112, sondern unter einem beliebigen schrägen Winkel auf die Gehäusewand 112 gestrahlt. An der Grenzfläche zwischen der Gehäusewand 112 und dem Dialysat oder dem Blut sowie an allen weiteren Grenzflächen im Innenvolumenbereich 120 der Dialysatorvorrichtung 100 findet Lichtbrechung statt. Wie dem Fachmann bekannt ist, hängt diese vom Brechungsindex der an den Grenzflächen vorhandenen Stoffen ab. Aufgrund ihrer Konzentration und ihrer starken optischen Wechselwirkungen haben die optischen Eigenschaften des Dialysats und des Bluts den größten Effekt auf die Lichtbrechung. Die Messung von Lichtbrechung kann somit zur Analyse von physikalischen Eigenschaften des Dialysats bzw. des Bluts herangezogen werden, z.B. zur Messung der Dichte.

Die Detektion der nach Lichtbrechung aus der Dialysatorvorrichtung 100 austretenden gebrochenen Lichtstrahlung kann ortsaufgelöst an einer von der Einstrahlstelle verschiedenen Stelle der Sensorvorrichtung 200 erfolgen. Der Ort der Detektion, z.B. durch selektive Auswahl aktiver Lichteintrittsöffnungen 256-x,y in der zweidimensionalen Anordnung 255 in Fig.3, und die Einfallsrichtung und -stelle des eingestrahlten Lichts können dabei aufeinander so abgestimmt werden, dass aus der Position des detektierten gebrochenen Lichts innerhalb der zweidimensionalen Anordnung 255 der Strahlungseintrittsöffnungen 256-x,y auf den bei der Lichtbrechung untergangenen Brechungswinkel zurückgeschlossen werden kann. Daraus kann auf den komplexen Brechungsindex und daraus wiederum auf die den Brechungsindex beeinflussenden physikalischen Eigenschaften der Flüssigkeiten im Dialysat bzw. im Blut, z.B. ihre Dichte, zurück geschlossen werden. Um auf den komplexen Brechungsindex zurückzuschliessen, wird bei einem, mehreren oder allen Ausführungsbeispielen die Ablenkung des Strahls und der Intensitätsverlust bei Durchgang durch das Medium (Absorption) erfasst. Der Realteil wird durch die Ablenkung bestimmt, der Imaginärteil durch Absorption. Aus den physikalischen Eigenschaften lassen sich medizinisch relevante Parameter ableiten, wie etwa die Hämatokritkonzentration im Blut.

Bei einem, mehreren oder allen Ausführungsbeispielen können auch Transmissionsmessungen an der Dialysatorvorrichtung durchgeführt werden. Dies wird im Folgenden näher dargelegt.

Wird die Blickrichtung eines Detektors in der Sensorvorrichtung 200 an einer Stelle platziert und ausgerichtet, auf die die eingestrahlte einfallende Lichtstrahlung gerichtet ist, so lässt sich aus der Dämpfung bzw. aus der Abnahme der Lichtstrahlung beim Durchlaufen eines Laufwegs im Innenvolumenbereich 120, etwa im Blutbereich 130 oder im Diaysatbereich 170, ein Absorptionskoeffizient entlang der von der Lichtstrahlung durchlaufenen Laufstrecke bestimmen. Wird die Lichtstrahlung selektiv bezüglich der Absorptionwellenlänge gemessen, so kann durch Auswahl der Messwellenlänge eine stoffspezifische Absorption gemessen und auf eine Konzentration des entsprechenden Stoffes in dem Raumbereich entlang der Laufstrecke des Lichts zurück geschlossen werden. Derartige Transmissions- bzw. Absorptionsmessungen ermöglichen Rückschlüsse auf die stoffliche Zusammensetzung des Dialysats bzw. des Bluts. Bei einem, mehreren oder allen Ausführungsbeispielen kann eine Messung von Streustrahlung an der Dialysatorvorrichtung durchgeführt werden. Dies wird im Folgenden näher dargelegt.

Mit in den Beispielen 1 und 2 beschriebenen Messgeometrien kann auch die in der Dialysatorvorrichtung 100 gestreute Lichtstrahlung analysiert werden. Dazu kann an einer beliebigen Position an der Dialysatorvorrichtung 100, die sich möglichst weder in der Ausbreitungsrichtung des eingestrahlten noch in der Ausbreitungsrichtung des gebrochenen noch in der Ausbreitungsrichtung des reflektierten Lichts befindet, die Intensität des gestreuten Lichts bestimmt werden. Vorzugsweise wird dabei das gestreute Licht an mehreren Stellen an der Gehäuseoberfläche 112 der Dialysatorvorrichtung gemessen, was z.B. mit der in Fig. 3 gezeigten Ausführungsform einer Sensorvorrichtung 200 mit einer zweidimensionalen Anordnung 255 von Lichteintrittsöffnungen 256-x,y möglich ist. Aus der Intensität des gestreuten Lichts lässt sich auf die Anwesenheit von Stoffen, z.B. bestimmten Molekülen, deren Abmessungen typischerweise bis zu 10-mal kleiner ist als die Wellenlänge des verwendeten Lichts, zurück schließen. Je größer die Konzentration derartiger Stoffe bzw. Strukturen, desto größer ist die Intensität der gestreuten Strahlung. Aus der räumlichen Verteilung der Intensität der gestreuten Strahlung in den unterschiedlichen Raumrichtungen lässt sich auch auf die Größe und die Form der Stoffe bzw. Strukturen (z.B. Moleküle) zurück schließen.

Ein Spezialfall von mit der Sensoreinrichtung 200 induzierbarer Lichtstreuung stellt die dynamische Lichtstreuung dar. Dabei wird eine stark monochromatische und stark gerichtete bzw. gebündelte Lichtquelle, wie etwa ein Laserstrahl, als Lichteinstrahlungseinrichtung 210 eingesetzt. Mittels einer zweidimensionalen Lichtdetektionsanordnung, wie etwa der zweidimensionalen Anordnung 255 der Lichteintrittsöffnungen 256-x,y in FIg. 3, kann die räumliche Verteilung des gestreuten Lichts gemessen werden. Die räumliche Verteilung wird unter Anderem durch Interferenz des gestreuten eingestrahlten Lichts beeinflusst, wobei Licht auf unterschiedlichen Laufwegen (Streupfaden) von dem einen eingestrahlten Lichtstrahl einen gleichen Punkt der zweidimensionalen (flächigen) Detektoreinrichtung erreicht. Aus der gemessenen zeitlichen Veränderung des mit der flächigen Detektoreinrichtung gemessenen Interferenzmusters lässt sich auf die Bewegung der streuenden Zentren zurück schließen. Sind die analysierten Streuzentren beispielsweise rote Blutkörperchen, so kann auf deren Geschwindigkeit zurück geschlossen werden, insbesondere auf die Geschwindigkeit ihrer (ungerichteten) Brownschen Bewegung, und daraus auf die Koagulationsneigung des Bluts in Kapillaren der Membran der Membranfiltereinrichtung 190 und darauf basierend können gegebenenfalls Änderungen von Parametern der Dialysetherapie eingeleitet werden.

Bei einem, mehreren oder allen Ausführungsbeispielen kann eine elektromagnetische Identifizierung einer in der Dialysetherapie verwendeten Dialysator- bzw. Komponente, z.B. einer Membranfiltereinrichtung durchgeführt werden. Dies wird im Folgenden näher dargelegt.

Elektromagnetische Strahlung kann auch eingesetzt werden, um die in einer Dialysetherapie an einem Patienten 10 verwendete Dialysatorvorrichtung 100 bzw. Membranfiltereinrichtung 190 automatisiert zu identifizieren. So kann die Dialysemaschine 300 Informationen über die in der Dialysetherapie eines Patienten 10 verwendete (z.B. mittels der Gehäusehalter 180, 182 gehaltene) und als Einmalartikel ausgebildete Dialysatorvorrichtung 100 oder Membranfiltereinrichtung 190 ermitteln, beispielsweise den Typ bzw. eine Seriennummer oder eine sonstige spezielle Ausgestaltung derselben, ohne dass eine die Therapie durchführende medizinische Fachkraft in möglicherweise fehlerbehafteter Weise Informationen bezüglich der Identifizierung der in der Dialysetherapie verwendeten Dialysatorvorrichtung 100 bzw. Membranfiltereinrichtung 190, z.B. mittels der Eingabe-/Ausgabeeinrichtung 310 der Dialysemaschine 300, einzugeben braucht.

In einer Ausführungsform ist zu diesem Zweck in der Sensorvorrichtung 200 z.B. ein Barcode-Scanner, ein Detektor oder eine Kamera (zum Erkennen eines Codes, z.B. einer Datamatrix bzw. eines 2D-Codes oder eines Fluoreszenzcodes) integriert, und die Sensorvorrichtung 200 wird so auf der Dialysatorvorrichtung 100 platziert, dass der Barcode-Scanner bzw. der Detektor oder die Kamera einen auf der als Einmalartikel ausgebildeten Dialysatorvorrichtung 100 oder Membranfiltervorrichtung 190 angebrachten oder aufgedruckten Code, z.B. einen Barcode bzw. Datamatrix bzw. 2D- oder 3D-Code oder einen Fluoreszenzcode lesen kann. Mit optischen Detektoren kann auch eine Farbcode-Identifizierung, etwa von an dem Einmalartikel angebrachten oder aufgedruckten Farbcode oder über eine farbige Ausgestaltung des Dialysatorgehäuses 110 oder über eine spektrometrische bzw. eine true-colour Lichtdetektoreinrichtung, und damit eine Identifizierung der in der Dialysetherapie verwendeten Dialysatorvorrichtung 100 oder Membranfiltereinrichtung 190 realisiert werden.

Alternativ zu einer auf optischer Detektion beruhenden Identifizierung durch die Sensoreinrichtung 200 kann eine auf der Messung von RF (Englisch: radio frequency) Strahlung basierte Kommunikation mit einem an der Dialysatorvorrichtung 100 oder an der Komponente wie etwa der Membranfiltereinrichtung 190 angebrachten RFID-Chip aufgenommen werden, und in einer dem Fachmann bekannten Weise von dem RFID-Chip gespeicherte, charakteristische Identifizierungsinformation abgerufen werden. Ein Vorteil der Identifizierung mittels RF-Strahlung ist, dass der in der Sensorvorrichtung 200 integrierte RF-Detektor sehr kurzreichweitig, z.B. induktiv, ausgelegt werden kann und auf diese Weise mit hoher Sicherheit nur der RFID-Chip derjenigen Dialysatorvorrichtung 100 gelesen wird, an der die Sensorvorrichtung 200 aktuell angebracht wird.

Die hierin beschriebene bzw. die in den beigefügten Patentansprüchen beanspruchten Ausführungsformen der Sensorvorrichtung 200 gemäß dem zweiten Aspekt der Erfindung und der Dialysatorvorrichtung 100 gemäß dem dritten Aspekt der Erfindung ermöglichen in einer für einen Fachmann offensichtlichen Weise folgende Vorteile zu erzielen:
1) Messungen von Informationen bezüglich eines Zustands, z.B. einer Dialysance, oder einer Stoffkonzentration, oder bezüglich einer Identifizierung einer als Einmalartikel ausgebildeten Dialysatorvorrichtung 100 oder Membranfiltereinrichtung 190 während des Betriebs in einer Dialysebehandlung eines Patienten 10.
2) Messungen mit vergleichsweise hoher zeitlicher Auflösung, z.B. in Zeitintervallen von einigen Sekunden bis Minuten, die während einer laufenden Dialysebehandlung therapeutische Eingriffe zur Optimierung der Behandlung ermöglichen.
3) Messungen, insbesondere berührungslos durchgeführt, von Information bezüglich des Zustands der Dialysatorvorrichtung 100 oder der Membranfilterienrichtung 190 direkt an der Dialysatorvorrichtung 100.
4) Die Sensorvorrichtung 200 ist wieder verwendbar und damit kosteneffizient im Hinblick darauf, dass die Dialysatorvorrichtung 100 oder zumindest die darin angeordnete Membranfiltereinrichtung 190 ein Einmalartikel ist. Dieser Vorteil ist solange valide, bis zukünftig eine etwaige in der Dialysatorvorrichtung 100 integrierte Sensorik kostengünstiger sein wird.

Nachfolgend werden beispielhafte Aspekte in durchnummerierter Form beschrieben:
Aspekt 1 betrifft ein System zur Erfassung einer Eigenschaft oder eines Zustands einer Dialysatorvorrichtung (100) oder einer Komponente derselben wie etwa einer Membranfiltereinrichtung (190) der Dialysatorvorrichtung (100) vor oder während des Betriebs der Dialysatorvorrichtung (100), z.B. bei einer Blutbehandlung eines Patienten (10), wobei das System folgendes umfasst:
   die Dialysatorvorrichtung (100) mit einem einen Innenvolumenbereich (120) umschließenden Gehäuse (110) und einer im Wesentlichen in dem Innenvolumenbereich (120) angeordneten Membranfiltereinrichtung (190), wobei das Gehäuse (110) für ein Signal, wie etwa ein Strahlungssignal, zumindest bereichsweise durchlässig sein kann, und
   eine Sensorvorrichtung (200), die mit dem Gehäuse (110) der Dialysatorvorrichtung (100) lösbar verbindbar ist und die eine Signalempfangseinrichtung (210, 212, 222, 224) umfasst, die dazu ausgebildet ist, mindestens ein Signal, wie etwa ein Strahlungssignal von dem Außen- oder Randbereich des Gehäuse oder aus dem Innenvolumenbereich (120) des Gehäuses (110) zu empfangen, wobei das Signal charakteristisch für die Dialysatorvorrichtung oder deren Zustand oder für die Blutbehandlung ist.
Aspekt 2. System nach Aspekt 1, das dazu ausgebildet ist, das empfangene Signal einer Auswerteeinrichtung (330) bereitzustellen, die auf der Grundlage des empfangenen Signals Daten erzeugt, die zum Steuern von Betriebsparametern einer Dialysemaschine (300) verwendet werden können.
Aspekt 3. System nach Aspekt 1 oder 2, bei dem die Signalempfangseinrichtung (210, 222, 224) dazu ausgebildet ist, Signale einer Strahlung zu empfangen oder zu detektieren, die ausgewählt ist aus einer Gruppe, die folgendes umfasst:
   a) elektromagnetische Strahlung des gesamten elektromagnetischen Spektrums, optional mit einer Wellenlänge im optischen Bereich wie etwa im Bereich ferninfraroten, infraroten (IR), nahinfraroten, sichtbaren und ultravioletten (UV) Lichts,
   b) elektromagnetische Strahlung mit einer Wellenlänge bzw. dieser entsprechenden Frequenz im Mikrowellenbereich, im Terahertzbereich, d.h. im Submillimeterstrahlungsbereich, oder im Bereich von Radiowellen, wie etwa in der RFID-Technologie verwendeten Radiowellen, und
   c) Ultraschall-Strahlung.
Aspekt 4. System nach Aspekt 1 oder 2, bei dem die Signalempfangseinrichtung (210) einen Empfänger für ein elektrisches Signal umfasst, wobei das elektrische Signal indikativ für eine zu messende Kapazität und/oder eine zu messende Induktivität ist, die charakteristisch für den Zustand bzw. die Identifizierung der Dialysatorvorrichtung (100) und/oder der Membranfiltereinrichtung (190) ist.
Aspekt 5. System nach Aspekt 3, bei dem die Sensorvorrichtung (200) eine Strahlungsaussendeeinrichtung (240) umfasst, die dazu ausgebildet ist, Strahlung, wie etwa Licht- oder Ultraschall-Strahlung, in den Innenvolumenbereich (120) des Gehäuses (110) der Dialysatorvorrichtung (100) auszusenden, und dass
   die Signalempfangseinrichtung (210, 222, 224) dazu ausgebildet ist, einen oder mehrere Parameter wie etwa die Intensität, Phase und/oder das Zeitverhalten von Strahlung, die von der Strahlungsaussendeeinrichtung (240) ausgesendet worden ist, zu messen, wobei die gemessene Strahlung optional das Ergebnis einer für den Zustand der Dialysatorvorrichtung (200) charakteristischen Wechselwirkung sein kann, die im Innenvolumenbereich (120) des Gehäuses (110) zwischen der Strahlung und einem oder mehreren der folgenden stattgefunden hat:
   - dem Dialysat bzw. dem Blut,
   - einem in dem Dialysat und/oder in dem Blut enthaltenen Stoff,
   - der Membranfiltereinrichtung (190) und
   - einem in bzw. an der Membranfiltereinrichtung (190) festgehaltenen Stoff, der aus dem Blut bzw. dem Dialysat entstammt,
   wobei zumindest ein Teil der wechselwirkenden Strahlung aus dem Innenvolumenbereich (120) zu der Signalempfangseinrichtung (210, 222, 224) gelangt ist, und
   wobei insbesondere die Wechselwirkung durch von der Strahlungsaussendeeinrichtung (240) in den Innenvolumenbereich (120) des Gehäuses (110) ausgesendeten Strahlung hergerufen worden ist.
Aspekt 6. System nach Aspekt 5, bei dem eine optische Wechselwirkung hervorgerufen wird, die ausgewählt ist aus einer Gruppe, die folgendes umfasst:
   - Reflektion von von der Strahlungsaussendeeinrichtung (240) ausgesendeter optischer Strahlung an einer Grenzfläche zwischen der Gehäusewand (112), beispielsweise dem Fensterbereich (114, 116), und dem Dialysat oder dem Blut,
   - Reflektion von von der Strahlungsaussendeeinrichtung (240) ausgesendeter optischer Strahlung an einer Grenzfläche zwischen der Membranfiltereinrichtung (190) und dem Dialysat oder dem Blut,
   - Transmission von von der Strahlungsaussendeeinrichtung (240) ausgesendeter optischer Strahlung mit einer Messwellenlänge, die von einem in dem Dialysat und/oder in dem Blut enthaltenen Stoff absorbiert werden kann und die auf ihrem Weg zu der Strahlungsempfangseinrichtung einen Strahlungslaufweg durch das Dialysat und/oder durch das Blut zurückgelegt hat,
   - Emission von Lumineszenz- oder Fluoreszenzstrahlung durch einen in dem Dialysat und/oder in dem Blut enthaltenen Stoff, wobei eine Lumineszenz- oder Fluoreszenzreaktion in diesem Stoff durch von der Strahlungsaussendeeinrichtung (240) ausgesendete optischer Strahlung hervorgerufen worden ist,
   - Brechung von von der Strahlungsaussendeeinrichtung (240) ausgesendeter optischer Strahlung an einer Grenzfläche zwischen der Gehäusewand (112), beispielsweise einem Fensterbereich (114, 116, 118), und dem Dialysat oder dem Blut, wobei die ausgesendete Strahlung unter einem zwischen 0° und 180° oder 0° und 90° liegenden Einfallswinkel auf der Grenzfläche auftrifft,
   - Streuung von von der Strahlungsaussendeeinrichtung (240) ausgesendeter optischer Strahlung an einem in dem Dialysat oder in dem Blut enthaltenen Stoff, einschließlich dynamischer Streuung von monochromatischer Laserstrahlung, und
   - Wechselwirkung von von der Strahlungsaussendeeinrichtung (240) ausgesendeter optischer Strahlung mit einer Identifizierungseinrichtung (192), die an der Dialysatorvorrichtung (100) oder an der Membranfiltereinrichtung (190) angebracht ist, wie etwa einem Lumineszenzcode, Barcode oder einem Farbencodefeld, die ein für die Identifizierung der Dialysatorvorrichtung (100) oder der Membranfiltereinrichtung (190) charakteristisches Identifizierungsmerkmal aufweist.
Aspekt 7. System nach Aspekt 3, bei dem die Dialysatorvorrichtung (100) oder die Membranfiltereinrichtung (190) eine passive Identifizierungseinrichtung (192), wie etwa ein Barcode, ein Farbencodefeld, einen Lumineszenzcode, also einen mit lumineszierendem Material aufgebrachten Code, oder einen passiven RFID Chip, umfasst, die ein für die Identifizierung der Dialysatorvorrichtung (100) oder der Komponente wie etwa der Membranfiltereinrichtung (190) charakteristisches Identifizierungsmerkmal aufweist, das durch Bestrahlen der Identifizierungseinrichtung (192) mit einer vorbestimmten elektromagnetischen Strahlung auslesbar ist,
   wobei die Sensorvorrichtung (200) eine Strahlungsaussendeeinrichtung (240) umfasst, die dazu ausgebildet ist, die vorbestimmte elektromagnetische Strahlung, insbesondere zu der Identifizierungseinrichtung (192), auszusenden, und dass
   die Signalempfangseinrichtung (210) eine Identifizierungsleseeinrichtung (212) umfasst, die dazu ausgebildet ist, einen für das Identifizierungsmerkmal indikativen Parameter der Strahlung zu detektieren und das Identifizierungsmerkmal zu bestimmen.
Aspekt 8. System nach Aspekt 3, bei dem die Dialysatorvorrichtung (100) oder die Membranfiltereinrichtung (190) eine aktive Identifizierungseinrichtung (192), wie etwa einen aktiven RFID Chip, umfasst, die elektromagnetische Strahlung aussenden kann, die ein für die Identifizierung der Dialysatorvorrichtung (100) oder der Membranfiltereinrichtung (190) charakteristisches Identifizierungsmerkmal aufweist, und
   bei dem die Signalempfangseinrichtung (210) eine Identifizierungsleseeinrichtung (212) umfasst, die dazu ausgebildet ist, die von der Identifizierungseinrichtung (192) ausgesendete elektromagnetische Strahlung zu detektieren und, etwa nach einer Demodulationsauswertung, das Identifizierungsmerkmal zu bestimmen.
Aspekt 9. System nach Aspekt 5 oder 6, bei dem die Strahlungsaussendeeinrichtung (240) mindestens eines oder mehrere der folgenden Merkmale umfasst:
   - eine Lichtquelle, die Licht in einem schmalbandigen Spektralbereich aussendet, wie etwa ein Laser oder eine LED,
   - eine Lichtleitfaser, die aus einer Licht in einem schmalbandigen Spektralbereich aussendenden Lichtquelle ausgesendetes Licht führt und einen Auskopplungsabschnitt aufweist, aus dem das Licht austritt,
   - eine Lichtquelle, die Licht in einem breitbandigen Spektralbereich aussendet, wie etwa eine Halogenlampe,
   - eine Lichtleitfaser, die aus einer Licht in einem breitbandigen Spektralbereich aussendenden Lichtquelle ausgesendetes Licht führt und einen Auskopplungsabschnitt aufweist, aus dem das Licht austritt.
Aspekt 10. System nach Aspekt 9, bei dem die Strahlungsaussendeeinrichtung (240) eine 1-dimensionale Anordnung (231, 233) oder eine 2-dimensionale Anordnung (236) von mehreren Strahlungsaustrittsbereichen (250) umfasst, wobei die Anordnung (231, 233, 235) im verbundenen Zustand der Sensorvorrichtung (200) und der Dialysatorvorrichtung (100) quer, schräg oder im Wesentlichen parallel zu einer Strömungsrichtung des Dialysats in dem Dialysatbereich (170) oder quer, schräg oder parallel zu der Strömungsrichtung des Bluts in dem Blutbereich (130) oder quer, schräg oder parallel zu einer Längsachse (102) oder entlang des Umfangs der Dialysatorvorrichtung (100) ausgerichtet ist.
Aspekt 11. System nach Aspekt 9 oder 10, bei dem die Sensorvorrichtung (200) eine Strahlungsmesseinrichtung (220) mit einer Messoptikeinrichtung, die eine Fokaltiefe und eine Fokalrichtung aufweist, und mit einem Lichtdetektor, der eine lichtsensitive Detektorfläche aufweist, umfasst, wobei die Messoptikeinrichtung dazu ausgebildet ist, einen Raumbereich aus dem Innenvolumenbereich (120) des Gehäuses (110) der Dialysatorvorrichtung (100) auf die Detektorfläche des Lichtdetektors abzubilden, wobei der abgebildete Raumbereich durch die Fokaltiefe und die Fokalrichtung definiert ist, und wobei die Messoptikeinrichtung so ausgebildet ist, dass ihre Fokaltiefe und Fokalrichtung vor der Inbetriebnahme oder während des Betriebs so verstellbar oder wählbar ist, dass der abgebildete Raumbereich im Bereich des Innenvolumenbereichs (120) wählbar ist.
Aspekt 12. System nach einem der Aspekte 9 bis 11, bei dem die Sensorvorrichtung (200) eine Strahlungsmesseinrichtung (220, 260) mit mindestens einem Strahlungseintrittsbereich (230, 232-x, 234-y, 236-x,y) und mindestens einem Lichtdetektor umfasst, der dazu ausgebildet ist, Strahlung, wie etwa Licht, die in den mindestens einen Strahlungseintrittsbereich (230, 232-x, 234-y, 236-x,y) eingetreten ist, zu detektieren und der aus einer Gruppe ausgewählt sein kann, die optional folgende Merkmale allein oder in beliebiger Kombination umfassen kann:
   eine oder mehrere Photodioden,
   einen oder mehrere Phototransistoren,
   einen oder mehrere CMOS-Lichtdetektoren,
   einen oder mehrere Photomultiplier,
   eine oder mehrere Avalanche-Photodioden,
   einen oder mehrere 1-dimensionale oder 2-dimensionale CCD-Sensoren,
   einen oder mehrere 1-dimensionale oder 2-dimensionale CMOS-Sensoren,
   eine oder mehrere 1-dimensionale Anordnungen (231) oder eine oder mehrere 2-dimensionale Anordnungen (235) einer Vielzahl von Photodioden, Phototransistoren, CMOS-Lichtdetektoren, Photomultiplier, Avalanche-Photodioden oder anderen Sensoren,
   einen oder mehrere andersgeartete Detektoren wie etwa MPPC (Multi Photon Pixel Counter).
Aspekt 13. System nach Aspekt 12, bei dem ein jeweiliger Strahlungseintrittsbereich (230, 232-x, 234-y, 236-x,y) einen Einkoppelbereich eines optischen Lichtleiters oder einer Lichtleitfaser umfasst, der/die dazu ausgebildet ist, die Strahlung zu einem dem Strahlungseintrittsbereich (230, 232-x, 234-y, 236-x,y) zugeordneten Lichtdetektor zu leiten.
Aspekt 14. System nach einem der vorhergehenden Aspekte, bei dem die Sensorvorrichtung (200) eine Strahlungsmesseinrichtung (220) mit mindestens einem Lichtdetektor und einer Wellenlängenauswahleinrichtung, wie etwa einem Monochromator, umfasst, wobei die Wellenlängenauswahleinrichtung dazu ausgebildet ist, einen schmalbandigen Wellenlängenbereich, der eine Nachweiswellenlänge umfasst, aus dem breitbandigeren Wellenlängenbereich der von der Strahlungsaussendeeinrichtung (24) ausgesendeten Strahlung, z.B. Licht, einstrahlungsseitig oder empfangsseitig so auszuwählen, dass eine optische Wechselwirkung, wie etwa Absorption oder Anregung von Lumineszenz- oder Fluoreszenzemission, mit einem ausgewählten Stoff, der sich im Betrieb an oder in der Dialysatorvorrichtung oder in dem Innenvolumenbereich (120) des Gehäuses (110) befindet, wie etwa einem Bestandteil des Dialysats und/oder des Bluts, erfolgt.
Aspekt 15. System nach einem der Aspekte 1 bis 14, bei dem die Sensorvorrichtung (200) dazu ausgebildet ist, mindestens einen der folgenden Parameter zu erfassen:
   im Blutbereich (30, 130) des Innenvolumenbereichs (120), Messen eines oder mehrerer für die Konzentration eines oder mehrerer Stoffe wie etwa eines urämischen Toxins indikativen Parameter, wie etwa die Absorption von Licht mit einer oder mehreren Messwellenlängen, die von dem Stoff wie etwa dem urämischen Toxin absorbiert werden,
   im Dialysatbereich (70, 170) des Innenvolumenbereichs (120), Messen eines für die Konzentration eines oder mehrerer Stoffe wie etwa eines urämischen Toxins indikativen Parameters, wie etwa die Absorption von Licht mit einer Messwellenlänge, das von dem Stoff absorbiert wird, und
   im Blutbereich (30, 130), Messen eines für eine physikalische Eigenschaft des Bluts, wie etwa Viskosität oder Hämatokrit-Konzentration, indikativen Parameters.
Aspekt 16. System nach einem der Aspekte 1 bis 15, bei dem das Gehäuse der Dialysatorvorrichtung (100) so ausgebildet ist, dass die Sensorvorrichtung (200) in einer oder mehreren vorbestimmten Positionen relativ zu dem Gehäuse (110) lösbar, z.B. formschlüssig oder mit Luftspalt, fixiert werden kann.
Aspekt 17. System nach Aspekt 16, bei dem das Gehäuse (110) an seiner Außenseite an einer oder mehreren Positionen erste Koppelbereiche (111, 111') aufweist, und dass die Sensorvorrichtung (200) einen zweiten Koppelbereich (201, 201') aufweist, der formschlüssig mit dem ersten Koppelbereich (111, 111') eingreifbar, z.B. andrückbar ausgebildet ist.
Aspekt 18. Sensorvorrichtung (200), die mit einem Gehäuse (110) einer Dialysatorvorrichtung (100) lösbar verbindbar ausgebildet ist und mindestens einen Sensor zur Erfassung eines aus dem Inneren des Gehäuses stammenden Signals aufweist, wobei durch die Sensorvorrichtung in einem mit der Dialysatorvorrichtung (100) verbundenen Zustand optional ein System gemäß einem der Aspekte 1 bis 17 ausbildbar ist.
Aspekt 19. Sensorvorrichtung (200) nach Aspekt 18, die mindestens einen Strahlungssender zur Aussendung von Strahlung auf die Oberfläche und/oder in das Innere der Dialysatorvorrichtung aufweist, wobei die Sensorvorrichtung optional wieder verwendbar ausgebildet ist.
Aspekt 20. Sensorvorrichtung (200) nach Aspekt 18 oder 19, bei der für die an dem Gehäuse (110) lösbar, z.B. formschlüssig befestigbare Ausbildung der Sensorvorrichtung (200) mindestens einer der folgenden Mechanismen vorgesehen ist:
   die Sensorvorrichtung (200) ist als eine Clipeinrichtung mit mindestens einem oder mehreren flexiblen Cliparmen oder Clipmanschetten (210, 210') ausgebildet, wobei der oder die Cliparme oder Clipmanschetten (210, 210') dazu ausgebildet sein können, das Gehäuse (110) in einem Winkelbereich von mehr als 180°, vorzugsweise mehr als 270° und noch mehr bevorzugt mehr als 320° zu umgreifen,
   die Sensorvorrichtung (200) umfasst einen Grundkörper und eine Clipeinrichtung mit mindestens einem oder zwei an dem Grundkörper befestigten elastisch-flexiblen Cliparmen oder an dem Grundkörper elastisch-flexibel angelenkten Cliparme, der oder die dazu ausgebildet sind, das Gehäuse (110) zumindest teilweise zu umgreifen oder mit diesem eine feste lösbare Verbindung einzugehen oder aufgrund ihrer elastisch-flexiblen Ausgestaltung oder Anlenkung das Gehäuse (110) clipartig zu umklammern,
   die Sensorvorrichtung (200) umfasst ein erstes Glied einer Verbindungseinrichtung, wie etwa eine Schwalbenschwanzeinrichtung, eine Schraube bzw. ein Klettband, und die Dialysatorvorrichtung (100) ein zweites Glied der Verbindungseinrichtung, wie etwa eine Schwalbenschwanzaufnahmeeinrichtung, ein zum Einschrauben der Schraube ausgebildetes Loch oder Schraubengewinde bzw. ein Gegenstück zu dem Klettband, wobei das erste Glied in das zweite Glied oder das zweite Glied in das erste Glied lösbar formschlüssig eingreifbar ausgebildet ist,
   die Sensorvorrichtung (200) wird mittels einer elastischen Spanneinrichtung, wie etwa einem Gummiring, an dem Gehäuse (110) festgespannt, wobei die Spanneinrichtung dazu ausgebildet ist, in einem elastisch gespannten Zustand die Sensorvorrichtung (200) und das Gehäuse (110) der Dialysatorvorrichtung (100) zu umzuspannen,
   die Sensorvorrichtung (100) umfasst einen Grundkörper und mindestens einen Arm oder ein Paar von an dem Grundkörper angelenkten oder mit dem Grundkörper elastisch-flexibel verbundenen Armen bzw. Manschetten, die jeweils einen distalen Endbereich mit einem dort vorgesehenen ersten Glied einer Haken- oder Eingreifeinrichtung aufweisen, wobei der oder die Arme bzw. Manschetten dazu ausgebildet sind, das Gehäuse (110) in einem Winkelbereich von mehr als 180°, vorzugsweise mehr als 270°, und mehr bevorzugt mehr als 320°, zu umgreifen, und eine elastisch spannbare Spanneinrichtung mit einem oder zwei gegenüberliegenden Endabschnitten, an denen jeweils ein zum lösbaren Eingreifen mit einem ersten Glied der Haken- oder Eingreifeinrichtung ausgebildetes zweites Glied einer Haken- oder Eingreifeinrichtung vorgesehen sein kann, wobei in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung (200) eingreifen kann, und
   die Sensorvorrichtung (200) ist dazu ausgebildet, beispielsweise in einer Vertiefung oder mit einem Vorsprung, in der Gehäusewand (112) der Dialysatorvorrichtung (100) lösbar integriert zu werden.
Aspekt 21. Dialysatorvorrichtung (100) mit einem einen Innenvolumenbereich (120) umschließenden Gehäuse (110) und einer im Wesentlichen in dem Innenvolumenbereich (120) angeordneten Membranfiltereinrichtung (190), wobei das Gehäuse (110) für ein Signal, wie etwa ein Strahlungssignal, zumindest bereichsweise durchlässig ist, und die Dialysatorvorrichtung (100) so ausgebildet ist, dass mit ihr eine Sensorvorrichtung nach einem der Ansprüche 18 bis 20 lösbar operativ verbindbar und in einem verbundenen Zustand ein System gemäß einem der Ansprüche 1 bis 17 ausbildbar ist.
Aspekt 22. Dialysatorvorrichtung (100) nach Aspekt 21, mit mindestens einem, wahlweise mehreren oder allen der folgenden Merkmale:
   das Gehäuse (100) umfasst eine insbesondere zylinderförmige Gehäusewand (112) mit mindestens einem für ein Signal durchlässigen Fensterbereich (114, 116, 118);
   die Membranfiltereinrichtung (190) kann in das Gehäuse (100) herausnehmbar eingebracht werden, ein Innengehäuse kann in dem Gehäuse (110) lösbar fixiert werden und umschließt die Membranfiltereinrichtung (190) im Wesentlichen,
   das Innengehäuse, insbesondere mit der darin angeordneten Membranfiltereinrichtung (190), kann in das Gehäuse (100) herausnehmbar eingebracht werden,
      zumindest die Membranfiltereinrichtung (190) ist als Einmalartikel ausgebildet,
   das Innengehäuse und die Membranfiltereinrichtung (190), oder die Dialysatorvorrichtung (100) insgesamt, sind als Einmalartikel ausgebildet
   der Innenvolumenbereich (120) umfasst einen im Betrieb von einem Dialysat durchströmbaren Dialysatbereich (170) und einen im Betrieb von Blut durchströmbaren Blutbereich (130), wobei der Dialysatbereich (170) von dem Blutbereich (130) durch die Membranfiltereinrichtung (190) räumlich getrennt sind,
   die Membranfiltereinrichtung (190) ist dazu ausgebildet, im Betrieb einen durch Konzentrationsgradienten von in dem Blut bzw. in dem Dialysat gelösten Stoffen getriebenen Stoffaustausch zwischen dem Blut und dem Dialysat zu ermöglichen,
   die Dialysatorvorrichtung ist als intelligenter Dialysator ausgebildet und / oder weist Schnittstellen zu einer externen Steuereinrichtung auf.
Aspekt 23. Dialysemaschine (300) zum Durchführen einer Dialysebehandlung an einem Patienten, mit einer Dialysatorvorrichtung (100) nach einem der Aspekte 21 oder 22 und einer Sensorvorrichtung (200) nach einem der Aspekte 18 bis 20, die optional dazu vorgesehen sind, ein System gemäß einem der Aspekte 1 bis 17 auszubilden oder zu einem derartigen System lösbar operativ verbunden zu werden.
Aspekt 24. Dialysemaschine (300) nach Aspekt 23, bei der die Dialysatorvorrichtung (100) zumindest einen Gehäusehalter (180, 182) umfasst, der separat ausgebildet oder mit der Dialysemaschine fest verbunden ist und dazu ausgebildet ist, mit der Dialysemaschine (300) zumindest im Betrieb mechanisch verbunden zu sein und die Dialysatorvorrichtung (100) lösbar zu fixieren.
Aspekt 25. Dialysemaschine (300) nach Aspekt 24, bei der die Sensorvorrichtung (200) in dem Gehäusehalter (180, 182) integriert ist.

### Bezugszeichenliste:

- 10: Patient
- 20: Blutkreislauf
- 22: arterieller Zugang
- 24: arterielles Schlauchsystem
- 26: arterielle Blutpumpe
- 28: Bluteinlass
- 32: Blutauslass
- 34: venöses Schlauchsystem
- 36: venöser Zugang
- 40: Dialysatkreislauf
- 42: Wasseraufbereitung
- 44: Bikarbonat-Konzentrat
- 46: Bikarbonat-Pumpe
- 48: Bikarbonat-Zufluss
- 54: Säure-Konzentrat
- 56: Säure-Pumpe
- 58: Säure-Zufluss
- 60: Bilanzierungseinrichtung - Dialysatzugang
- 62, 62': Dialysatzufuhr-Zufluss
- 64: Flusspumpe-Dialysatzugang
- 66: Bypass-Ventil
- 68: Bypass-Leitung
- 72: Ventil Dialysator Eingang
- 74: Dialysateinlass
- 76: Dialysatauslass
- 78: Ventil Dialysator Ausgang
- 80: Bilanzierungseinrichtung - Dialysatausfluss
- 82, 82': Dialysatausfluss
- 84: Flusspumpe-Dialysatausfluss
- 86: Dialysatabfuhr
- 100: Dialysatorvorrichtung
- 102: Längsachse
- 104: Umfang
- 110: Gehäuse
- 111, 111': erster Koppelbereich
- 112: Gehäusewand
- 114: erster Fensterbereich
- 116: zweiter Fensterbereich
- 118: dritter Fensterbereich
- 120: Innenvolumenbereich
- 130: Blutbereich
- 170: Dialysatbereich
- 180: erster Gehäusehalter
- 182: zweiter Gehäusehalter
- 190: Membranfiltereinrichtung
- 192: Identifizierungseinrichtung
- 200: Sensorvorrichtung
- 210, 210': Clipmanschette
- 211, 211': zweiter Koppelbereich
- 210: Signalempfangseinrichtung
- 212: Identifizierungsleseeinrichtung
- 220: Strahlungsmesseinrichtung
- 222: erster Strahlungsdetektor
- 224: zweiter Strahlungsdetektor
- 230: Strahlungseintrittsbereich
- 231: 1-dimensionale Anordnung
- 232-x: Strahlungseintrittsbereich
- 233: 1-dimensionale Anordnung
- 234-y: Strahlungseintrittsbereich
- 235: 2-dimensionale Anordnung
- 236-x,y: Strahlungseintrittsbereich
- 240: Strahlungsaussendeeinrichtung
- 242: erste Strahlungsquelle
- 244: zweite Strahlungsquelle
- 250: Strahlungsaustrittsbereich
- 251: 1-dimensionale Anordnung
- 252-x: Strahlungsaustrittsbereich
- 253: 1-dimensionale Anordnung
- 254-y: Strahlungsaustrittsbereich
- 255: 2-dimensionale Anordnung
- 256-x,y: Strahlungsaustrittsbereich

- 300: Dialysemaschine
- 302: Maschinengehäuse
- 304, 304': Gehäuserollen
- 310: Eingabe-/Ausgabeeinrichtung
- 320: Steuereinheit
- 330: Datenverarbeitungs- bzw. Auswerteeinheit
- 340: Speichereinheit
- 360: Datenbussystem
- 362: Eingabe-/Ausgabe-Datenleitung
- 364: Steuerleitung
- 366: Betriebsdatenleitung
- 368: Datenspeicherleitung
- 370: Dialysatorvorrichtungssignalleitung
- 372: Sensorvorrichtungssignalleitung

## Patentansprüche

1. Identifikationsvorrichtung einer Dialysatorvorrichtung (100) oder einer Komponente derselben, wobei
die Komponente eine Membranfiltereinrichtung (190) ist, wobei
die Dialysatorvorrichtung (100) und/oder eine daran oder in ihr angeordnete Membranfiltereinrichtung (190) eine passive oder eine aktive Identifizierungseinrichtung (192) umfassen, die ein für die Identifizierung der Dialysatorvorrichtung (100) und/oder Membranfiltereinrichtung (190) charakteristisches Identifikationsmerkmal aufweist,
**gekennzeichnet dadurch, dass**
die Identifikationsvorrichtung eine Sensorvorrichtung (200) aufweist, wobei
die Sensorvorrichtung (200) als eine Clipeinrichtung mit mindestens einem flexiblen Cliparm oder Clipmanschette (210) ausgebildet ist, die dafür angepasst ist, um die in der Identifizierungseinrichtung (192) enthaltenen Identifikationsmerkmale auszulesen und hierfür mit einem Gehäuse (110) der Dialysatorvorrichtung (100) oder Membranfiltereinrichtung (190) direkt lösbar verbindbar ist und die eine Signalempfangseinrichtung (210, 212, 220, 222, 224) umfasst, die dazu ausgebildet ist, mindestens ein Signal von dem Gehäuse, einem Außen- oder Randbereich des Gehäuses, oder aus einem Innenvolumenbereich (120) des Gehäuses (110) zu empfangen, wobei
die Dialysatorvorrichtung (100) oder Membranfiltereinrichtung (190) so ausgebildet ist, dass die Sensorvorrichtung (200) ein solches Signal erfassen oder messen kann, welches, entsprechend den Identifikationsmerkmalen, charakteristisch für die Dialysatorvorrichtung (100) oder Membranfiltereinrichtung (190) ist, so dass eine Identifizierung der Dialysatorvorrichtung (100) oder Membranfiltereinrichtung (190) erfolgen kann und wobei
die Identifikationsvorrichtung weiter eine Auswerteeinrichtung (330) aufweist, die dazu ausgebildet ist, auf der Grundlage des empfangenen Signals Daten zu erzeugen, die zur Identifizierung oder zur Identifizierung und Steuerung von Betriebsparametern einer Dialysemaschine (300) verwendbar sind.

2. Vorrichtung nach Anspruch 1 , bei der die Signalempfangseinrichtung (210, 220, 222, 224) dazu ausgebildet ist, Signale einer Strahlung zu empfangen oder zu detektieren, die ausgewählt ist aus einer Gruppe, die folgendes umfasst:
a) elektromagnetische Strahlung des gesamten elektromagnetischen Spektrums,
b) elektromagnetische Strahlung mit einer Wellenlänge bzw. dieser entsprechenden Frequenz im Mikrowellenbereich, im Terahertzbereich, d.h. im Submillimeterstrahlungsbereich, oder im Bereich von Radiowellen, und
c) Ultraschall-Strahlung.

3. Vorrichtung nach Anspruch 1 oder 2, bei der die Sensorvorrichtung (200) eine Strahlungsaussendeeinrichtung (240) umfasst, die dazu ausgebildet ist, Strahlung, auf das Gehäuse oder in den Innenvolumenbereich (120) des Gehäuses (110) der Dialysatorvorrichtung (100) auszusenden, und
die Signalempfangseinrichtung (210, 220, 222, 224) dazu ausgebildet ist, einen oder mehrere Parameter von empfangener Strahlung zu messen.

4. Vorrichtung nach einem der vorhergehenden Ansprüche, bei der die Dialysatorvorrichtung (100) oder die Komponente (190) eine passive Identifizierungseinrichtung (192) umfasst, die ein für die Identifizierung der Dialysatorvorrichtung (100) oder der Komponente charakteristisches Identifizierungsmerkmal aufweist, das durch Bestrahlen der Identifizierungseinrichtung (192) mit einer vorbestimmten elektromagnetischen Strahlung auslesbar ist,
wobei die Sensorvorrichtung (200) eine Strahlungsaussendeeinrichtung (240) umfasst, die dazu ausgebildet ist, die vorbestimmte elektromagnetische Strahlung zu der Identifizierungseinrichtung (192), auszusenden, und dass
die Signalempfangseinrichtung (210) eine Identifizierungsleseeinrichtung (212) umfasst, die dazu ausgebildet ist, einen für das Identifizierungsmerkmal indikativen Parameter der Strahlung zu detektieren und das Identifizierungsmerkmal zu bestimmen.

5. Vorrichtung nach einem der vorhergehenden Ansprüche, bei der die Dialysatorvorrichtung (100) oder die Komponente (190) eine aktive Identifizierungseinrichtung (192), wie etwa einen aktiven RFID Chip, umfasst, die elektromagnetische Strahlung aussenden kann, die ein für die Identifizierung der Dialysatorvorrichtung (100) oder der Komponente (190) charakteristisches Identifizierungsmerkmal aufweist, und
bei dem die Signalempfangseinrichtung (210) eine Identifizierungsleseeinrichtung (212) umfasst, die dazu ausgebildet ist, die von der Identifizierungseinrichtung (192) ausgesendete elektromagnetische Strahlung zu detektieren und das Identifizierungsmerkmal zu bestimmen.

6. Vorrichtung nach Anspruch 2 oder 3, bei der die Strahlungsaussendeeinrichtung (240) mindestens eines oder mehrere der folgenden Merkmale umfasst:
- eine Lichtquelle, die Licht in einem schmalbandigen Spektralbereich aussendet,
- eine Lichtleitfaser, die aus einer Licht in einem schmalbandigen Spektralbereich aussendenden Lichtquelle ausgesendetes Licht führt und einen Auskopplungsabschnitt aufweist, aus dem das Licht austritt,
- eine Lichtquelle, die Licht in einem breitbandigen Spektralbereich aussendet,
- eine Lichtleitfaser, die aus einer Licht in einem breitbandigen Spektralbereich aussendenden Lichtquelle ausgesendetes Licht führt und einen Auskopplungsabschnitt aufweist, aus dem das Licht austritt.

7. Vorrichtung nach einem der vorhergehenden Ansprüche, bei der die Sensorvorrichtung (200) eine Strahlungsmesseinrichtung (220, 260) mit mindestens einem Strahlungseintrittsbereich (230, 232-x, 234-y, 236-x,y) und mindestens einem Lichtdetektor umfasst, der dazu ausgebildet ist, Strahlung, wie etwa Licht, die in den mindestens einen Strahlungseintrittsbereich (230, 232-x, 234-y, 236-x,y) eingetreten ist, zu detektieren und der aus einer Gruppe ausgewählt sein kann, die folgende Merkmale allein oder in beliebiger Kombination umfassen kann:
eine oder mehrere Photodioden,
einen oder mehrere Phototransistoren,
einen oder mehrere CMOS-Lichtdetektoren,
einen oder mehrere Photomultiplier,
eine oder mehrere Avalanche-Photodioden,
einen oder mehrere 1-dimensionale oder 2-dimensionale CCD-Sensoren,
einen oder mehrere 1-dimensionale oder 2-dimensionale CMOS-Sensoren,
eine oder mehrere 1-dimensionale Anordnungen (231) oder eine oder mehrere 2-dimensionale Anordnungen (235) einer Vielzahl von Photodioden, Phototransistoren, CMOS-Lichtdetektoren, Photomultiplier, Avalanche-Photodioden oder anderen Sensoren,
einen oder mehrere andersgeartete Detektoren wie etwa MPPC (Multi Photon Pixel Counter).

8. Vorrichtung nach einem der vorhergehenden Ansprüche, bei der die Sensorvorrichtung (200) eine Strahlungsmesseinrichtung (220) mit mindestens einem Lichtdetektor und einer Wellenlängenauswahleinrichtung, wie etwa einem Monochromator, umfasst, wobei die Wellenlängenauswahleinrichtung dazu ausgebildet ist, einen schmalbandigen Wellenlängenbereich, der eine Nachweiswellenlänge umfasst, aus dem breitbandigeren Wellenlängenbereich der von der Strahlungsaussendeeinrichtung (24) ausgesendeten Strahlung, z.B. Licht, einstrahlungsseitig oder empfangsseitig so auszuwählen, dass eine optische Wechselwirkung, wie etwa Absorption oder Anregung von Lumineszenz- oder Fluoreszenzemission, mit einem ausgewählten Stoff, der sich im Betrieb an oder in der Dialysatorvorrichtung oder in dem Innenvolumenbereich (120) des Gehäuses (110) befindet erfolgt.

9. Vorrichtung nach einem der vorhergehenden Ansprüche, bei der das Gehäuse der Dialysatorvorrichtung (100) so ausgebildet ist, dass die Sensorvorrichtung (200) in einer oder mehreren vorbestimmten Positionen relativ zu dem Gehäuse (110) lösbar, z.B. formschlüssig oder mit Luftspalt, fixiert werden kann.

10. Vorrichtung nach Anspruch 9, bei der das Gehäuse (110) an seiner Außenseite an einer oder mehreren Positionen erste Koppelbereiche (111, 111') aufweist, und die Sensorvorrichtung (200) einen zweiten Koppelbereich (201, 201') aufweist, der formschlüssig mit dem ersten Koppelbereich (111, 111') eingreifbar, z.B. andrückbar ausgebildet ist.

11. Vorrichtung nach einem der vorhergehenden Ansprüche, bei der die Sensorvorrichtung (200) in einem Gehäusehalter (180, 182) zur Halterung einer Dialysatorvorrichtung (100) integriert ist.

12. Vorrichtung gemäß einem der Ansprüche 1 bis 11, wobei die Sensorvorrichtung (200) eine lösbare Befestigungseinrichtung aufweist, die derart ausgelegt ist, dass sie mit einem Gehäuse (110) einer Dialysatorvorrichtung (100) lösbar verbindbar ist und mindestens einen Sensor zur Erfassung eines von dem Außenbereich oder aus dem Inneren des Gehäuses stammenden Signals aufweist.

13. Vorrichtung (200) nach Anspruch 12, mit mindestens einem Strahlungssender zur Aussendung von Strahlung auf die Oberfläche und/oder in das Innere der Dialysatorvorrichtung, wobei die Sensorvorrichtung wieder verwendbar ausgebildet ist.

14. Vorrichtung (200) nach Anspruch 12 oder 13, mit mindestens einem der folgenden Mechanismen für die lösbare, z.B. formschlüssig befestigbare Anbringung der Sensorvorrichtung (200) an dem Gehäuse (110):
die Sensorvorrichtung (200) ist als eine Clipeinrichtung mit mindestens einem oder mehreren flexiblen Cliparmen oder Clipmanschetten (210, 210') ausgebildet, wobei der oder die Cliparme oder Clipmanschetten (210, 210') dazu ausgebildet sein können, das Gehäuse (110) in einem Winkelbereich von mehr als 180° zu umgreifen,
die Sensorvorrichtung (200) umfasst einen Grundkörper und eine Clipeinrichtung mit mindestens einem oder zwei an dem Grundkörper befestigten elastisch-flexiblen Cliparmen oder an dem Grundkörper elastisch-flexibel angelenkten Cliparme, der oder die dazu ausgebildet sind, das Gehäuse (110) zumindest teilweise zu umgreifen oder mit diesem eine feste lösbare Verbindung einzugehen oder aufgrund ihrer elastisch-flexiblen Ausgestaltung oder Anlenkung das Gehäuse (110) clipartig zu umklammern,
die Sensorvorrichtung (200) umfasst ein erstes Glied einer Verbindungseinrichtung und die Dialysatorvorrichtung (100) ein zweites Glied der Verbindungseinrichtung, wobei das erste Glied in das zweite Glied oder das zweite Glied in das erste Glied lösbar formschlüssig eingreifbar ausgebildet ist,
die Sensorvorrichtung (200) wird mittels einer elastischen Spanneinrichtung an dem Gehäuse (110) festgespannt, wobei die Spanneinrichtung dazu ausgebildet ist, in einem elastisch gespannten Zustand die Sensorvorrichtung (200) und das Gehäuse (110) der Dialysatorvorrichtung (100) zu umzuspannen,
die Sensorvorrichtung (100) umfasst einen Grundkörper und mindestens einen Arm oder ein Paar von an dem Grundkörper angelenkten oder mit dem Grundkörper elastisch-flexibel verbundenen Armen bzw. Manschetten, die jeweils einen distalen Endbereich mit einem dort vorgesehenen ersten Glied einer Haken- oder Eingreifeinrichtung aufweisen, wobei der oder die Arme bzw. Manschetten dazu ausgebildet sind, das Gehäuse (110) in einem Winkelbereich von mehr als 180° zu umgreifen, und eine elastisch spannbare Spanneinrichtung mit einem oder zwei gegenüberliegenden Endabschnitten, an denen jeweils ein zum lösbaren Eingreifen mit einem ersten Glied der Haken- oder Eingreifeinrichtung ausgebildetes zweites Glied einer Haken- oder Eingreifeinrichtung vorgesehen sein kann, wobei in einem elastisch gespannten Zustand der Spanneinrichtung ein jeweiliges zweites Glied am Endabschnitt der Spanneinrichtung mit einem ersten Glied an den Endbereichen der Arme bzw. Manschetten der Sensorvorrichtung (200) eingreifen kann, und
die Sensorvorrichtung (200) ist dazu ausgebildet, in der Gehäusewand (112) der Dialysatorvorrichtung (100) lösbar integriert zu werden.

15. Vorrichtung gemäß einem der Ansprüche 1 bis 14 als Bestandteil einer Dialysemaschine (300) zum Durchführen einer Dialysebehandlung an einem Patienten, die eine Dialysatorvorrichtung (100) und eine Sensorvorrichtung (200) aufweist.

16. Vorrichtung nach Anspruch 15, mit einem Gehäusehalter (180, 182), der separat von der Dialysatorvorrichtung (100) ausgebildet oder mit der Dialysemaschine fest verbunden ist, und der dazu ausgebildet ist, die Dialysatorvorrichtung (100) lösbar zu fixieren.

17. Vorrichtung nach Anspruch 16, bei der die Sensorvorrichtung (200) in dem Gehäusehalter (180, 182) integriert ist.

## Claims

1. An identifying device of a dialyzer apparatus (100) or a component thereof, wherein
the component is a membrane filter device (190), wherein
the dialyzer apparatus (100) and/or a membrane filter device (190) disposed thereon or in it comprises a passive or active identification unit (192) that comprises an identification feature that is characteristic for the identification of the dialyzer apparatus (100) and/or the membrane filter device (190),
wherein the identifying device comprises a sensor device (200), and **characterized in that**
the sensor device (200) is designed as a clip unit having at least one flexible clip arm or clip cuff (210) adapted for reading the identification features included in the identification unit (192) and therefor may be directly connected in a removable fashion to a housing (110) of the dialyzer apparatus (100) or the membrane filter device (190) and that comprises a signal receiving unit (210, 212, 220, 222, 224) that is designed to receive at least one signal from the housing, from the outside or edge region of the housing, or from the internal volume portion (120) of the housing (110), wherein
the dialyzer apparatus (100) or the membrane filter device (190) is designed such that the sensor device (200) can detect or measure such a signal which is, corresponding to the identification features, characteristic for the dialyzer apparatus or the membrane filter device (190), such that an identification of the dialyzer apparatus (100) or the membrane filter device (190) may occur, and wherein
the identifying device further comprises an analysis unit (330) that is designed to generate data on the basis of the received signal that may be used for identification or for identification and controlling the operating parameters of a dialysis machine (300).

2. The device according to Claim 1, wherein the signal receiving unit (210, 220, 222, 224) is designed to receive or detect signals of a radiation selected from a group that comprises the following:
a) electromagnetic radiation of the entire electromagnetic spectrum,
b) electromagnetic radiation having a wavelength or a frequency corresponding thereto in the microwave range, in the terahertz range, i.e., in the submillimeter radiation range, or in the range of radio waves, and
c) ultrasound radiation.

3. The device according to Claim 1 or 2, with the sensor device (200) comprising a radiation transmitting unit (240) that is designed to transmit radiation onto the housing or into the internal volume portion (120) of the housing (110) of the dialyzer apparatus (100), and with
the signal receiving unit (210, 220, 222, 224) being designed to measure one or more parameters of received radiation.

4. The device according to one of the previous Claims in which the dialyzer apparatus (100) or the component (190) comprises a passive identification unit (192) that comprises an identification feature characteristic for the identification of the dialyzer apparatus (100) or the component and that may be read by irradiating the identification unit (192) with a predetermined electromagnetic radiation,
with the sensor device (200) comprising a radiation transmitting unit (240) that is designed to transmit the predetermined electromagnetic radiation to the identification unit (192) and
with the signal receiving unit (210) comprising an identification reading unit (212) that is designed to detect a parameter of the radiation that is indicative of the identification feature and to determine the identification feature.

5. The device according to one of the previous Claims, in which the dialyzer apparatus (100) or the component (190) comprises an active identification unit (192), such as an active RFID chip, that is able to transmit electromagnetic radiation having an identification feature that is characteristic for the identification of the dialyzer apparatus (100) or the component (190), and
in which the signal receiving unit (210) comprises an identification reading unit (212) that is designed to detect the electromagnetic radiation transmitted by the identification unit (192) and determine the identification feature.

6. The device according to Claim 2 or 3, in which the radiation transmitting unit (240) comprises at least one or more of the following features:
- a light source that transmits light in a narrow-band spectral range,
- a light-conducting fiber that guides light transmitted by a light source emitting light in a narrow-band spectral range and comprises a decoupling segment from which the light exits,
- a light source that transmits light in a wide-band spectral range,
- a light-conducting fiber that guides light transmitted by a light source emitting light in a wide-band spectral range and comprises a decoupling segment from which the light exits.

7. The device according to one of the previous Claims, wherein the sensor device (200) comprises a radiation measuring unit (220, 260) having at least one radiation entrance region (230, 232-x, 234-y, 236-x,y) and at least one light detector that is designed to detect radiation, such as light, that has entered into the at least one radiation entrance region (230, 232-x, 234-y, 236-x,y) and that may be selected from a group that may include the following features alone or in any desired combination:
one or more photodiodes,
one or more phototransistors,
one or more CMOS light detectors,
one or more photomultipliers,
one or more avalanche photodiodes,
one or more one-dimensional or two-dimensional CCD sensors,
one or more one-dimensional or two-dimensional CMOS sensors,
one or more one-dimensional arrangements (231) or one or more two-dimensional arrangements (235) of a plurality of photodiodes, phototransistors, CMOS light detectors, photomultipliers, avalanche photodiodes, or other sensors,
one or more detectors of another type such as, for example, MPPCs (multi photon pixel counters).

8. The device according to one of the previous Claims, in which the sensor device (200) comprises a radiation measuring unit (220) having at least one light detector and a wavelength selection unit, such as a monochromator, with the wavelength selection unit being designed to select a narrow-band wavelength range including a reference wavelength range from the wider-band wavelength range of the radiation emitted by the radiation transmitting unit (24), for example, light, on the transmitting side or on the receiving side in such a way that an optical interaction, such as absorption or excitement of luminescent or fluorescent emissions, occurs with a selected material located during operation on or in the dialyzer apparatus or in the internal volume portion (120) of the housing (110).

9. The device according to one of the previous Claims, wherein the housing of the dialyzer apparatus (100) is designed in such a way that the sensor device (200) may be fixed in a detachable fashion in one or more predetermined positions relative to the housing (110), for example, in a positive fit or with clearance.

10. The device according to Claim 9, wherein the housing (110) comprises first coupling regions (111, 111') at one or more positions on its exterior and the sensor device (200) comprises a second coupling region (201, 201') that is designed to engage in a positive fit with the first coupling region (111, 111'), for example, in a pressable fashion.

11. The device according to one of the previous Claims, wherein the sensor device (200) is integrated into a housing holder (180, 182) for holding a dialyzer apparatus.

12. The device according to one of Claims 1 to 11, wherein the sensor device (200) comprises a releasable fastening unit that is designed in such a way that it may be detachably connected to a housing (110) of a dialyzer apparatus (100) and comprises at least one sensor for detecting a signal originating from the exterior or from the interior of the housing.

13. The device (200) according to Claim 12, having at least one radiation transmitter for transmitting radiation onto the surface and/or into the interior of the dialyzer apparatus, with the sensor device being designed in a reusable fashion.

14. The device (200) according to Claim 12 or 13, having at least one of the following mechanisms for attaching the sensor device (200) to the housing (110) in a removable, for example positive-fitting, fashion:
the sensor device (200) is embodied as a clip unit having at least one or more flexible clip arms or clip cuffs (210, 210'), where the clip arms or clip cuffs (210, 210') may be designed to encompass the housing (110) in an angular range of more than 180°,
the sensor device (200) comprises a base body and a clip unit having at least one or two elastically flexible clip arms attached to the base body or elastically flexible clip arms articulated on the base body designed to encompass the housing (110) at least partially or to enter into a fixed, removable connection therewith or, due to its elastically flexible design or articulation, to encompass the housing (110) in a clip-like fashion,
the sensor device (200) comprises a first member of a connector unit and the dialyzer apparatus (100) comprises a second member of the connector unit, with the first member being designed to engage with the second member or with the second member being designed to engage with the first member in a removable positive fit,
the sensor device (200) is clamped on the housing (110) by means of an elastic clamping unit with the clamping unit being designed to encompass the sensor device (200) and the housing (110) of the dialyzer apparatus (100) in its elastically clamped state,
the sensor device (100) comprises a base body and at least one arm or a pair of arms or cuffs articulated on the base body or connected to the base body in an elastically flexible fashion, each comprising a distal end region having a first member of a hook or engaging unit provided thereon, with the arm or the arms and/or cuffs being designed to encompass the housing (110) in an angular range of more than 180° and an elastically tensible clamping unit having one or two end sections opposite one another, on each of which a second member of a hook or engaging unit is provided that is designed to detachably engage a first member of the hook or engaging unit, with each second member on the end section of the clamping unit in an elastically clamped state being able to engage with a first member on the end regions of the arms and/or clamps of the sensor device (200), and
the sensor device (200) is designed to be integrated into the housing wall (112) of the dialyzer apparatus (100) in a removable fashion.

15. The device according to one of Claims 1 to 14 as a component of a dialysis machine (300) for conducting a dialysis treatment on a patient, having a dialyzer apparatus (100) and a sensor device (200).

16. The device according to Claim 15, having a housing holder (180, 182) that is designed separately from the dialyzer apparatus (100) or that is connected in a fixed manner to the dialysis machine and that is designed to fix the dialyzer apparatus (100) in a removable fashion.

17. The device according to Claim 16, wherein the sensor device (200) is integrated into the housing holder (180, 182).

## Revendications

1. Dispositif d'identification d'un dispositif de dialyse (100) ou d'un composant de celui-ci, dans lequel
le composant est un système de filtre à membrane (190), dans lequel
le dispositif de dialyse (100) et/ou un système de filtre à membrane (190) agencé sur ou dans celui-ci comprennent un système d'identification (192) passif ou actif qui présente un élément d'identification caractéristique pour l'identification du dispositif de dialyse (100) et/ou du système de filtre à membrane (190), dans lequel
le dispositif d'identification présente un dispositif de capteur (200),
et **caractérisé en ce que**
le dispositif de capteur (200) est conçu comme un système de clip comportant au moins un bras de clip flexible ou un manchon à clip (210) qui est adapté pour lire les éléments d'identifications contenus dans le système d'identification (192) et peut être relié à cette fin avec un boîtier (110) du dispositif de dialyse (100) ou du système de filtre à membrane (190) de façon directement détachable et qui comprend un système de réception de signal (210, 212, 220, 222, 224) qui est conçu pour recevoir au moins un signal du boîtier, d'une région extérieure ou périphérique du boîtier ou d'une région de volume intérieur (120) du boîtier (110), dans lequel
le dispositif de dialyse (100) ou le système de filtre à membrane (190) est conçu de telle sorte que le dispositif de capteur (200) puisse saisir ou mesurer un tel signal qui est caractéristique du dispositif de dialyse (100) ou du système de filtre à membrane (190) selon les éléments d'identification, de telle sorte qu'une identification du dispositif de dialyse (100) ou du système de filtre à membrane (190) puisse avoir lieu et dans lequel
le dispositif d'identification présente en outre un système d'analyse (330) qui est conçu pour générer des données sur la base du signal reçu, qui sont utilisables pour l'identification ou pour l'identification et la commande de paramètres de fonctionnement d'une machine de dialyse (300).

2. Dispositif selon la revendication 1, dans lequel le dispositif de réception de signal (210, 220, 222, 224) est conçu pour recevoir ou détecter des signaux d'un rayonnement qui est choisi dans le groupe comprenant :
a) un rayonnement électromagnétique du spectre électromagnétique complet,
b) un rayonnement électromagnétique présentant une longueur d'onde ou une fréquence correspondant à celle-ci dans la plage des micro-ondes, dans la plage des téta-hertz, c'est-à-dire dans la plage de rayonnement submillimétrique ou dans la plage d'ondes radio, et
c) un rayonnement d'ultrasons.

3. Dispositif selon la revendication 1 ou 2, dans lequel le dispositif de capteur (200) comprend un système émetteur de rayonnement (240) qui est conçu pour émettre un rayonnement sur le boîtier ou dans la région de volume intérieur (120) du boîtier (110) du dispositif de dialyse (100), et
le système de réception de signal (210, 220, 222, 224) est conçu pour mesurer un ou plusieurs paramètres du rayonnement reçu.

4. Dispositif selon l'une des revendications précédentes, dans lequel le dispositif de dialyse (100) ou le composant (190) comprend un système d'identification passif (192) qui présente un élément d'identification caractéristique pour l'identification du dispositif de dialyse (100) ou du composant qui peut être lu par exposition du système d'identification (192) avec un rayonnement électromagnétique prédéterminé,
dans lequel le dispositif de capteur (200) comprend un système d'émission de rayonnement (240) qui est conçu pour émettre le rayonnement électromagnétique prédéterminé au système d'identification (192), et en ce que
le système de réception de signal (210) comprend un système de lecture d'identification (212) qui est conçu pour détecter un paramètre de rayonnement indicatif pour l'élément d'identification et pour déterminer l'élément d'identification.

5. Dispositif selon l'une des revendications précédentes, dans lequel le dispositif de dialyse (100) ou le composant (190) comprend un système d'identification actif (192) tel qu'une puce RFID active qui peut émettre un rayonnement électromagnétique qui présente un élément d'identification caractéristique pour l'identification du dispositif de dialyse (100) ou du composant (190), et
dans lequel le système de réception de signal (210) comprend un système de lecture d'identification (212) qui est conçu pour détecter le rayonnement électromagnétique émis par le système d'identification (192) et pour déterminer l'élément d'identification.

6. Dispositif selon la revendication 2 ou 3, dans lequel le système d'émission de rayonnement (240) comprend au moins un ou plusieurs des éléments suivants :
- une source de lumière qui émet de la lumière dans une plage spectrale à bande étroite,
- une fibre optique qui conduit la lumière émise à partir d'une source de lumière qui émet de la lumière dans une plage spectrale à bande étroite et présente un segment de couplage duquel sort la lumière,
- une source de lumière qui émet de la lumière dans une plage spectrale à bande large,
- une fibre optique qui conduit de la lumière émise à partir d'une source de lumière qui émet de la lumière dans une plage spectrale à bande large et présente un segment de couplage duquel sort la lumière.

7. Dispositif selon l'une des revendications précédentes, dans lequel le dispositif de capteur (200) comprend un système de mesure de rayonnement (220, 260) comportant au moins une plage d'entrée de rayonnement (230, 232-x, 234-y, 236-x,y) et au moins un photodétecteur qui est conçu de façon à détecter un rayonnement tel qu'une lumière qui est entrée dans l'au moins une plage d'entrée de rayonnement (230, 232-x, 234-y, 236-x,y) et peut être choisi dans un groupe qui peut comprendre les éléments suivants seuls ou en combinaisons quelconques :
une ou plusieurs photodiodes,
un ou plusieurs phototransistors,
un ou plusieurs photodétecteurs CMOS,
un ou plusieurs photomultiplicateurs,
une ou plusieurs photodiodes à avalanche,
un ou plusieurs capteurs CCD monodimensionnels ou bidimensionnels,
un ou plusieurs capteurs CMOS monodimensionnels ou bidimensionnels,
un ou plusieurs agencement monodimensionnels (231) ou un ou plusieurs agencements bidimensionnels (235) d'une multiplicité de photodiodes, phototransistors, photodétecteurs CMOS, photomultiplicateurs, photodiodes à avalanche ou autres capteurs,
un ou plusieurs détecteurs différents tels qu'un MPPC (Multi Photon Pixel Counter).

8. Dispositif selon l'une des revendications précédentes, dans lequel le dispositif de capteur (200) comprend un système de mesure de rayonnement (220) comportant au moins un photodétecteur et un système de sélection de longueur d'onde tel qu'un monochromateur, dans lequel le système de sélection de longueur d'onde est conçu pour sélectionner, au niveau du rayonnement ou de la réception, une plage de longueur d'onde à bande étroite qui comprend une longueur d'onde de détection, à partir de la plage de longueur d'onde à bande large du rayonnement émis par le système d'émission de rayonnement (24), par exemple de la lumière, de telle sorte qu'il s'effectue une interaction optique comme par exemple une absorption ou une excitation d'émission de luminescence ou de fluorescence avec une substance choisie qui se trouve en fonctionnement sur ou dans le dispositif de dialyse ou dans la région de volume intérieur (120) du boîtier (110).

9. Dispositif selon l'une des revendications précédentes, dans lequel le boîtier du dispositif de dialyse (100) est conçu de telle sorte que le dispositif de capteur (200) peut être fixé de façon détachable dans une ou plusieurs positions prédéterminées par rapport au boîtier (110), par exemple par coopération de forme ou avec un espace d'air.

10. Dispositif selon la revendication 9, dans lequel le boîtier (110) présente sur son côté extérieur sur une ou plusieurs positions, des premières régions de couplage (111, 111') et le dispositif de capteur (200) présente une deuxième région de couplage (201, 201') qui peut être en prise par coopération de forme avec la première région de couplage (111, 111'), par exemple qui peut être pressée.

11. Dispositif selon l'une des revendications précédentes, dans lequel le dispositif de capteur (200) est intégré dans un support de boîtier (180, 182) pour supporter un dispositif de dialyse (100).

12. Dispositif selon l'une des revendications 1 à 11, dans lequel le dispositif de capteur (200) présente un système de fixation détachable qui est configuré de telle sorte qu'il puisse être relié à un boîtier (110) d'un dispositif de dialyse (100) et présente au moins un capteur pour la saisie d'un signal provenant de la région extérieure ou de l'intérieur du boîtier.

13. Dispositif (200) selon la revendication 12, comportant au moins un émetteur de rayonnement pour émettre un rayonnement sur la surface et/ou à l'intérieur du dispositif de dialyse, le dispositif de capteur étant conçu de façon à être réutilisable.

14. Dispositif (200) selon la revendication 12 ou 13, comportant au moins l'un des mécanismes suivants pour le montage détachable, par exemple pouvant être fixé par coopération de forme du dispositif de capteur (200) au boîtier (110) :
le dispositif de capteur (200) est conçu comme un système de clip comportant au moins un ou plusieurs bras de clip flexibles ou manchons à clip (210, 210'), dans lequel le ou les bras de clip ou les manchons à clip (210, 210') peuvent être conçus pour entourer le boîtier (110) dans une plage angulaire supérieure à 180°,
le dispositif de capteur (200) comprend un corps de base et un système de clip comportant au moins un ou deux bras de clip élastiques-flexibles fixés au corps de base ou bras de clip articulés élastiques-flexibles sur le corps de base qui sont conçus pour enserrer au moins partiellement le boîtier (110) ou pour former avec celui-ci une liaison solide détachable ou pour serrer le boîtier (110) à la manière d'un clip, compte tenu de leur configuration élastique-flexible ou de leur articulation,
le dispositif de capteur (200) comprend un premier membre d'un système de liaison et le dispositif de dialyse (100) comprend un deuxième membre du système de liaison, dans lequel le premier membre est conçu de façon à pouvoir être en prise par coopération de forme, de façon détachable, dans le deuxième membre, ou le deuxième membre dans le premier membre,
le dispositif de capteur (200) est tendu au moyen d'un système de tension élastique sur le boîtier (110), dans lequel le système de tension est conçu de façon à couvrir dans un état tendu élastiquement, le dispositif de capteur (200) et le boîtier (110) du dispositif de dialyse (100),
le dispositif de capteur (100) comprend un corps de base et au moins un bras ou une paire de bras ou de manchons articulés avec le corps de base ou reliés de façon élastique-flexible au corps de base, qui présentent respectivement une région terminale distale comportant un premier membre prévu à ce niveau d'un système d'accrochage ou de mise en prise, dans lequel le ou les bras ou manchons sont conçus de façon à couvrir le boîtier (110) dans une plage angulaire supérieure à 180° et un système de tension pouvant être tendu élastiquement comportant une ou deux sections terminales opposées sur lesquelles respectivement, un deuxième membre d'un système d'accrochage ou de mise en prise conçu pour la mise en prise détachable avec un premier membre du système d'accrochage ou de mise en prise peut être prévu, dans lequel dans un état tendu élastiquement du système de tension, un deuxième membre respectif peut être mis en prise sur la section terminale du système de tension avec un premier membre sur les régions terminales des bras ou manchons du dispositif de capteur (200), et
le dispositif de capteur (200) est conçu pour être intégré de façon détachable dans la paroi du boîtier (112) du dispositif de dialyse (100).

15. Dispositif selon l'une des revendications 1 à 14 comme composant d'une machine de dialyse (300) pour la réalisation d'un traitement de dialyse chez un patient, qui présente un dispositif de dialyse (100) et un dispositif de capteur (200).

16. Dispositif selon la revendication 15, comportant un support de boîtier (180, 182) qui est conçu séparément du dispositif de dialyse (100) ou est relié fixement à la machine de dialyse et qui est conçu pour fixer de façon détachable le dispositif de dialyse (100).

17. Dispositif selon la revendication 16, dans lequel le dispositif de capteur (200) est intégré dans le support du boîtier (180, 182).
